# EUROPEAN PATENT APPLICATION

(11) **EP 4 039 676 A1**
(43) Date of publication of application: **10.08.2022**
(21) Application number: 20873051.5
(22) Date of filing: 05.10.2020
(51) Int. Cl.: C07D 263/56, C07D 263/58, A61K 31/421, A61P 9/00, A61P 13/12, A61K 8/49, A61Q 19/08, A23L 33/10

(54) **COMPOUND FOR INDUCING EXPRESSION OF ANTI-AGING GENE KLOTHO AND USE THEREOF**

(30) Priority: 02.10.2019 KR 20190122305
(71) Applicant: Klotho Sciences, Gyeonggi-do 13605 (KR)
(72) Inventor: JUNG, Dong Ju, Cheonan-si, Chungcheongnam-do 31166 (KR)
(74) Representative: J A Kemp LLP
(86) International application number: PCT/KR2020/013480
(87) International publication number: WO 2021/066608

(57) **Abstract**

The present invention relates to a compound for inducing the expression of the anti-aging gene klotho and a preparation method therefor. The compound represented by Chemical Formula 1, according to the present invention, has an excellent effect of increasing the expression level of the klotho gene which is a gene associated with aging, and thus can be useful for a pharmaceutical composition, cosmetic composition or food composition for preventing skin aging, inhibiting cellular senescence or preventing, treating alleviating a vascular aging-induced disease or a renal disease.

## Description

### [Technical Field]

The present invention relates to a compound for inducing the expression of the anti-aging gene klotho and a use thereof.

### [Background Art]

The fact that there may be genes capable of regulating aging in animals became known through senescence-accelerated mice (SAM) reported in 1981. These mice, which were accidentally made while crossing AKR/J series mice, aged much faster than mice of the same family, and these mice were confirmed to have mutations in several genes. Later, the discovery of a single group of aging-related genes was reported in the 1990s. The reported genes were genes that expressed an enzyme called DNA helicase in the RecQ family. It has been reported that, when mutations occur in these genes, the result that premature aging occurs or cancer is produced, which is known to be caused by affecting DNA repair. A single gene associated with aging is the klotho gene, which was reported in 1997. The klotho gene was accidentally discovered while creating an animal model for transgenic hypertension mice, but in mice that could not express this gene, premature aging occurred and their lives were shortened. More interestingly, the lifespan of mice that later increased the expression of this gene increased by 20.0 to 30.8% in males and 18.8 to 19.0% in females. This became the first opportunity to inform the world that the lifespan of mice can be increased or decreased depending on the expression of a single gene. In addition, the base sequence of the klotho gene was very similar among animals, and it was reported that it was about 98% identical between mice and humans. This indicates that lifespan can be regulated according to the expression of the klotho gene in humans as well.

In humans, the klotho gene is located on chromosome 13 and produces a membrane protein with a base sequence similar to β-glucosidase. The protein klotho is mainly expressed in renal tubular epithelial cells and brain choroid plexus, and has been reported to be expressed in some parathyroid glands. The klotho gene is a gene associated with various aging phenotypes, and in mice lacking the klotho gene, a syndrome similar to the aging process such as shortened lifespan, decreased activity, growth retardation, atherosclerosis, arterial calcification, osteoporosis, immature reproductive organs, infertility, skin atrophy, and emphysema occurs. In klotho mutant mice, atherosclerosis similar to Monckeberg type arteriosclerosis caused by aging in humans is observed in all arteries from the aorta to the arterioles, and angiogenesis and vasculogenesis are impaired.

The expression of klotho mRNA is significantly higher in kidney tissue than in other tissues, but the expression of klotho mRNA is decreased in the kidneys of mice in a disease model of hypertension, type 2 diabetes, diabetic nephropathy, and chronic renal failure. In mice with reduced klotho expression, the production of nitric oxide, which is a relaxation factor derived from vascular endothelium, decreases, and when the klotho gene is injected into Otsuka Long-Evans Tokushima fatty rat (OLETF) mice, which simultaneously have risk factors for many cardiovascular diseases, using a viral gene carrier, endovascular dysfunction is alleviated, the production of NO is increased, and blood pressure is lowered by suppressing vascular thickening and fibrosis. In addition, the klotho gene also affects glucose and insulin metabolism in mice, and statin, which is a representative therapeutic agent for hypercholesterolemia, increases the expression of klotho mRNA in renal proximal tubule cells. In mice with reduced klotho expression, osteopenia of low bone turnover states occurs due to impaired differentiation of both osteoblasts and osteoclasts, which is similar to the characteristics of bone loss and senile osteoporosis according to an increase in age in humans. Furthermore, in klotho mutant mice, abnormal elongation of the trabecular bone at the epiphysis area and abnormal trabecular bone tissue on microcomputerized tomography are observed, which is due to the disorder of the bone resorption process. Changes in clinical phenotypes caused by mutations in the klotho gene observed in humans are diverse. The functional variant of klotho (KL-VS) modification with mutations in three sites of the klotho gene exon2 is associated with lipid metabolism, blood pressure, lifespan, cognitive function, coronary artery disease and cerebrovascular disease, and microsatellite polymorphism and single base genetic polymorphism of the klotho gene are associated with bone density, and it has also been reported that the single base genetic polymorphism of the klotho gene in healthy adult women is associated with cardiovascular disease risk factors and bone density. Recently, the association between the klotho gene and Alzheimer's disease has also been reported in several papers. In Alzheimer dementia mouse models, it has been reported that overexpression of klotho increases the lifespan of mice by 30% and inhibits a reduction in cognitive function. Furthermore, it was observed that the production of amyloid beta protein in the brain was reduced by 50% by klotho expression. In humans, the expression level of klotho is inversely proportional to the progression of Alzheimer's disease, and a report was submitted that the klotho protein reduces the amount of inflammatory cytokines in the blood of patients with Alzheimer's disease.

Efforts have been continuously made to develop a material capable of inducing the expression of the klotho gene, which has such a clear senescence inhibitory effect. Among known materials, those reported to be able to induce the expression of klotho include rapamycin, vitamin D, statin, and the like. In 2012, a research team at Boston University reported three compounds that they found by screening for compounds capable of inducing the expression of the klotho gene using a library of 150,000 compounds in total. The present inventors selected a compound called compound H, which has a structure that is highly likely to be developed as a pharmaceutical, among the compounds, confirmed through actual experiments that the compound can express the klotho gene in cells, and published the research results on its mechanism of action in a paper. Since then, the present inventors have conducted experiments to analyze the structure of compound H (Comparative Example 1), found the structural characteristics of the compound capable of inducing klotho expression, and based on this, made a new compound whose activity was increased by 10 times or more.

Aging is an inevitable, gradual biological process that causes dysfunction and destruction of almost all tissues and organs, ultimately leading to death. The aging of the human body, for example, is associated with a decrease in cellular function and may lead to the development of various diseases. Aging is thought to be due to an interaction between genetic and acquired factors and is generally characterized by an increase in aging, a quantitative and qualitative decrease in stem cells, and an abnormal structure at the tissue level.

Thus, the present inventors synthesized a compound that enhances the expression of the klotho gene, and confirmed that the expression of the klotho gene is further improved compared to the previously known compounds, thereby completing the present invention.

### [Disclosure]

### [Technical Problem]

An object of the present invention is to provide a compound represented by Chemical Formula 1 or a pharmaceutically acceptable salt thereof.

Another object of the present invention is to provide a method for preparing the compound represented by Chemical Formula 1.

Still another object of the present invention is to provide a composition for inhibiting cellular senescence, including the compound represented by Chemical Formula 1 or a pharmaceutically acceptable salt thereof.

Yet another object of the present invention is to provide a cosmetic composition for preventing or alleviating skin aging, including the compound represented by Chemical Formula 1 or a pharmaceutically acceptable salt thereof.

Yet another object of the present invention is to provide a health functional food composition for preventing or alleviating skin aging, including the compound represented by Chemical Formula 1 or a pharmaceutically acceptable salt thereof.

Yet another object of the present invention is to provide a health food composition for preventing or alleviating skin aging, including the compound represented by Chemical Formula 1 or a pharmaceutically acceptable salt thereof.

Yet another object of the present invention is to provide a pharmaceutical composition for preventing or treating a vascular aging-induced disease, including the compound represented by Chemical Formula 1 or a pharmaceutically acceptable salt thereof.

Yet another object of the present invention is to provide a health functional food composition for preventing or alleviating a vascular aging-induced disease, including the compound represented by Chemical Formula 1 or a pharmaceutically acceptable salt thereof.

Yet another object of the present invention is to provide a health food composition for preventing or alleviating a vascular aging-induced disease, including the compound represented by Chemical Formula 1 or a pharmaceutically acceptable salt thereof.

Yet another object of the present invention is to provide a pharmaceutical composition for preventing or treating a renal disease, including the compound represented by Chemical Formula 1 or a pharmaceutically acceptable salt thereof.

Yet another object of the present invention is to provide a health functional food composition for preventing or alleviating a renal disease, including the compound represented by Chemical Formula 1 or a pharmaceutically acceptable salt thereof.

Yet another object of the present invention is to provide a health food composition for preventing or alleviating a renal disease, including the compound represented by Chemical Formula 1 or a pharmaceutically acceptable salt thereof.

### [Technical Solution]

To achieve the objects,
the present invention provides a compound represented by the following Chemical Formula 1 or a pharmaceutically acceptable salt thereof. 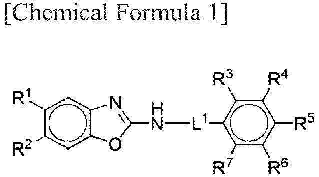 (in Chemical Formula 1,
L¹ is a single bond or 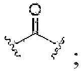
R¹ and R² are each -H, -OH, a C₁₋₁₀ straight or branched alkyl, or a C₆₋₈ arylamide, wherein the aryl of the arylamide may be substituted with one or more of a halogen, -NO₂ and a C₁₋₁₀ straight or branched alkyl halide;
R¹ and R² may form a C₆₋₈ aryl with a carbon atom to which they are linked; and
R³, R⁴, R⁵, R⁶ and R⁷ are each -H, a halogen, -NO₂ or a C₁₋₁₀ straight or branched alkyl).

Further, the present invention provides a method for preparing a compound represented by Chemical Formula 1A, as shown in the following Reaction Scheme 1, the method including:
obtaining Compound 4 by dissolving Compound 2 in an organic solvent, and then adding Compound 3 thereto, and reacting the resulting mixture at 10 to 50°C for 12 to 20 hours (Step 1); and
obtaining Compound 1A by adding an organic solvent in which Compound 4 obtained in Step 1 is dissolved dropwise to an organic solvent in which potassium superoxide is dissolved, and then reacting the resulting mixture at 15 to 30°C for 10 to 16 hours (Step 2): 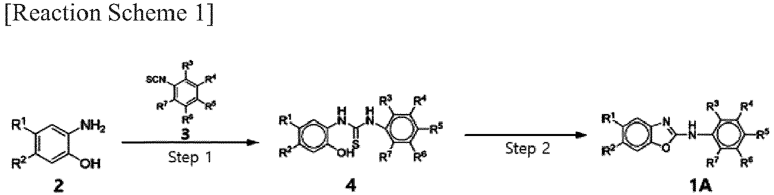
(in Reaction Scheme 1,
R¹, R², R³, R⁴, R⁵, R⁶ and R⁷ are the same as defined in Chemical Formula 1 of claim 1, and
Compound 1A is included in Chemical Formula 1 of claim 1).

Furthermore, the present invention provides a method for preparing a compound represented by Chemical Formula 1B, as shown in the following Reaction Scheme 2, the method including:
obtaining Compound 6 by dissolving Compound 5 in an organic solvent, and then adding Compound 3 thereto, and reacting the resulting mixture at 10 to 50°C for 12 to 20 hours (Step 1);
obtaining Compound 7 by adding an organic solvent in which Compound 6 obtained in Step 1 is dissolved dropwise to an organic solvent in which potassium superoxide is dissolved, and then reacting the resulting mixture for 12 to 24 hours (Step 2); and
obtaining Compound 1B by dissolving Compound 7 in an organic solvent, adding boron tribromide (BBr₃) thereto, and then reacting the resulting mixture at room temperature for 20 to 28 hours (Step 3): 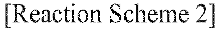 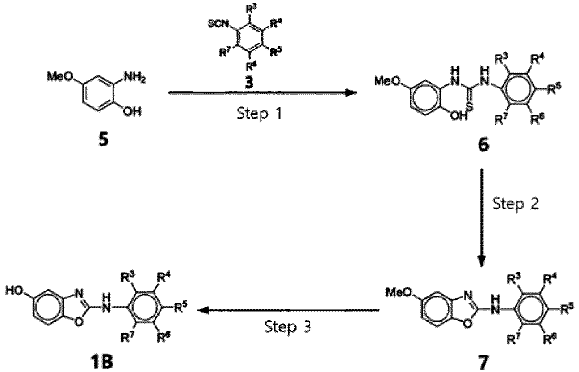 (in Reaction Scheme 2,
   R³, R⁴, R⁵, R⁶ and R⁷ are the same as defined in Chemical Formula 1 of claim 1, and
   Compound 1B is included in Chemical Formula 1 of claim 1).

Further, the present invention provides a method for preparing a compound represented by Chemical Formula 1C, as shown in the following Reaction Scheme 3, the method including:
obtaining Compound 9 by dissolving Compound 8, carbon disulfide, iodomethane and sodium hydride in an organic solvent, and then reacting the resulting solution at 10 to 50°C for 2 to 8 hours (Step 1); and
obtaining Compound 1C by dissolving Compound 9 and Compound 2 in an organic solvent, and then reacting the resulting solution for 2 to 8 hours (Step 2): 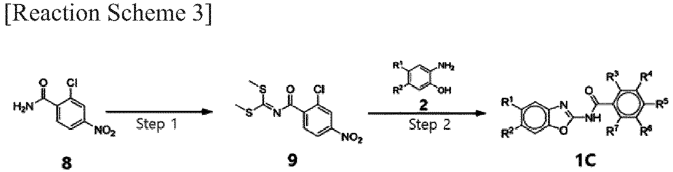 (in Reaction Scheme 3,
   R¹, R², R³, R⁴, R⁵, R⁶ and R⁷ are the same as defined in Chemical Formula 1 of claim 1, and
   Compound 1C is included in Chemical Formula 1 of claim 1).

Furthermore, the present invention provides a method for preparing a compound represented by Chemical Formula ID, as shown in the following Reaction Scheme 4, the method including:
obtaining Compound 11 by dissolving Compound 10 and Compound 3 in an organic solvent, and then reacting the resulting solution at 10 to 50°C for 20 to 28 hours (Step 1);
obtaining Compound 12 by adding an organic solvent in which Compound 11 obtained in Step 1 is dissolved dropwise to an organic solvent in which potassium superoxide is dissolved, and then reacting the resulting mixture at 10 to 50°C for 12 to 24 hours (Step 2);
obtaining Compound 13 by adding Compound 12 together with a catalyst to an organic solvent, and then injecting hydrogen gas thereinto, and reacting the resulting mixture at 10 to 50°C for 12 to 20 hours (Step 3); and
obtaining Compound ID by dissolving Compound 13 and Compound 14 in an organic solvent, and then reacting the resulting solution at 10 to 50°C for 12 to 24 hours (Step 4): 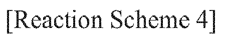 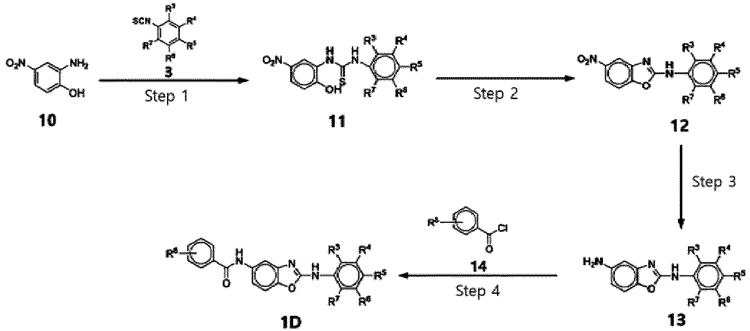 (in Reaction Scheme 4,
   R³, R⁴, R⁵, R⁶ and R⁷ are the same as defined in Chemical Formula 1 of claim 1;
   R⁸ is one or more of a halogen, -NO₂ and a C₁₋₁₀ straight or branched alkyl halide; and
   Compound ID is included in Chemical Formula 1 of claim 1).

In addition, the present invention provides a composition for inhibiting cellular senescence, including the compound represented by Chemical Formula 1 or a pharmaceutically acceptable salt thereof.

Furthermore, the present invention provides a cosmetic composition for preventing or alleviating skin aging, including the compound represented by Chemical Formula 1 or a pharmaceutically acceptable salt thereof.

Further, the present invention provides a health functional food composition for preventing or alleviating skin aging, including the compound represented by Chemical Formula 1 or a pharmaceutically acceptable salt thereof.

Furthermore, the present invention provides a health food composition for preventing or alleviating skin aging, including the compound represented by Chemical Formula 1 or a pharmaceutically acceptable salt thereof.

In addition, the present invention provides a pharmaceutical composition for preventing or treating a vascular aging-induced disease, including the compound represented by Chemical Formula 1 or a pharmaceutically acceptable salt thereof.

Further, the present invention provides a pharmaceutical composition for preventing or treating a vascular aging-induced disease, including the compound represented by Chemical Formula 1 or a pharmaceutically acceptable salt thereof, and an angiotensin II receptor blocker.

Furthermore, the present invention provides a health functional food composition for preventing or treating a vascular aging-induced disease, including the compound represented by Chemical Formula 1 or a pharmaceutically acceptable salt thereof.

In addition, the present invention provides a health food composition for preventing or treating a vascular aging-induced disease, including the compound represented by Chemical Formula 1 or a pharmaceutically acceptable salt thereof.

Furthermore, the present invention provides a pharmaceutical composition for preventing or alleviating a renal disease, including the compound represented by Chemical Formula 1 or a pharmaceutically acceptable salt thereof.

Further, the present invention provides a health functional food composition for preventing or alleviating a renal disease, including the compound represented by Chemical Formula 1 or a pharmaceutically acceptable salt thereof.

Furthermore, the present invention provides a health food composition for preventing or alleviating a renal disease, including the compound represented by Chemical Formula 1 or a pharmaceutically acceptable salt thereof.

### [Advantageous Effects]

The compound represented by Chemical Formula 1, according to the present invention, has an excellent effect of increasing the expression level of the klotho gene, which is a gene associated with aging, and thus can be useful for a pharmaceutical composition, cosmetic composition or food composition for preventing skin aging, inhibiting cellular senescence or preventing, alleviating or treating a vascular aging-induced disease or a renal disease.

### [Description of Drawings]

FIG. 1A illustrates the results of luciferase expression experiments using a reporter gene including a promoter from the start site of the human klotho gene of Comparative Examples 1 to 4 to the front of 1.7 kbp.
FIG. 1B illustrates the results of luciferase expression experiments using a reporter gene including a promoter from the start site of the human klotho gene of Comparative Examples 1 to 4 to the front of 240 bp.
FIG. 2 illustrates the results of luciferase expression experiments using a reporter gene including a promoter included up to -2.1 kb upstream of the human klotho gene of Examples 1 to 6.
FIG. 3 illustrates the results of luciferase expression experiments using a reporter gene including a promoter included up to -2.1 kb upstream of the human klotho gene of Examples 1 to 3.
FIG. 4 is a result of confirming the mRNA expression level of the klotho (KL) gene in Examples 1 and 2 by RT-PCR.
FIG. 5 is a result of confirming the expression of the klotho gene in RPTEC cells treated with the compounds of Examples 1 and 2 and Examples 7 to 10.
FIG. 6 is a result of confirming cytotoxicity in HK2 cells treated with the compounds of Examples 1, 9 and 10.
FIG. 7A schematically illustrates a protocol for creating a disease model by treating HK-2 cells (human kidney cells) with cisplatin.
FIG. 7B is a result of analyzing the degree of the expression level of a klotho protein in HK-2 cells obtained according to the protocol of FIG. 7A.
FIG. 8A schematically illustrates a protocol for obtaining an experimental sample by injecting a KS1 compound into a unilateral ureter obstruction animal model.
FIG. 8B is a result of analyzing the degree of renal hypertrophy using an experimental sample obtained according to the protocol of FIG. 8A.
FIG. 9 is a result of confirming the changes in the nucleus and cytoplasm in a unilateral ureter obstruction model through H&E staining.
FIG. 10 is a result of confirming the degree of fibrosis progression in a unilateral ureter obstruction model through Sirius Red staining.
FIG. 11 is a result of confirming the degree of apoptosis in a unilateral ureter obstruction model through TUNEL staining.
FIG. 12 is a result of analyzing changes in klotho protein expression in a unilateral ureter obstruction model.
FIG. 13 is a result of analyzing changes in MMP-9 protein expression in a unilateral ureter obstruction model.
FIG. 14 is a result of analyzing the effect of reducing hypertension of a KS1 compound.
FIG. 15 is a result of analyzing the effect of the KS 1 compound on inhibiting cellular senescence.

### [Modes of the Invention]

Hereinafter, the present invention will be described in detail.

### Compound or pharmaceutically acceptable salt thereof

The present invention provides a compound represented by the following Chemical Formula 1 or a pharmaceutically acceptable salt thereof. 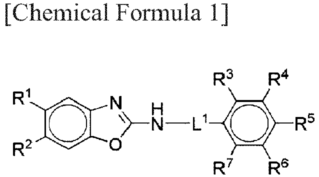 In Chemical Formula 1,
L¹ is a single bond or 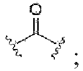
R¹ and R² are each -H, -OH, a C₁₋₁₀ straight or branched alkyl, or a C₆₋₈ arylamide, wherein the aryl of the arylamide may be substituted with one or more of a halogen, -NO₂ and a C₁₋₁₀ straight or branched alkyl halide;
R¹ and R² may form a C₆₋₈ aryl with a carbon atom to which they are linked; and
R³, R⁴, R⁵, R⁶ and R⁷ may each be -H, a halogen, -NO₂ or a C₁₋₁₀ straight or branched alkyl.

In an exemplary embodiment according to the present invention,
L¹ is a single bond or 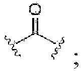
R¹ and R² are each -H, -OH, a C₁₋₅ straight or branched alkyl, or a C₆₋₇ arylamide, wherein the aryl of the arylamide may be substituted with one or more of a halogen, -NO₂ and a C₁₋₅ straight or branched alkyl halide;
R¹ and R² may form a C₆₋₇ aryl with a carbon atom to which they are linked; and
R³, R⁴, R⁵, R⁶ and R⁷ may each be -H, a halogen, -NO₂ or a C₁₋₅ straight or branched alkyl.

In an exemplary embodiment according to the present invention,
L¹ is a single bond or 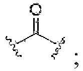
R¹ and R² are each -H, -OH, -CH₃, or phenylamide, wherein the phenyl of the phenylamide may be substituted with one or more of -Cl, -NO₂ and -CH₂Cl;
R¹ and R² may form a phenyl with a carbon atom to which they are linked; and
R³, R⁴, R⁵, R⁶ and R⁷ may each be -H, -F, -Cl, -NO₂ or -CH₂CH₃.

In an exemplary embodiment according to the present invention,
L¹ is a single bond or 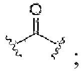
R¹ is -H, -OH, -CH₃, 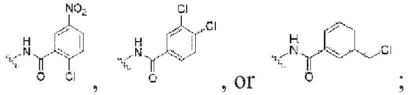
R² is -H;
R¹ and R² may form a phenyl with a carbon atom to which they are linked;
R³ is -H or -Cl;
R⁴ is -H, -F or -Cl;
R⁵ is -F, -Cl, -NO ₂, or -CH₂CH₃;
R⁶ is -H; and
R⁷ may be -H.

Preferred examples of the compound represented by Chemical Formula 1 according to the present invention include the following compound group.
1) N-(benzo[d]oxazol-2-yl)-2-chloro-4-nitrobenzamide;
2) 8-methyl-2-[N-(3,4-dichlorophenyl)]aminobenzoxazole;
3) 2-((3,4-dichlorophenyl)amino)benzo[d]oxazol-5-ol;
4) N-(2-(4-ethylphenylamino)benzo[d]oxazol-5-yl)-2-chloro-5-nitrobenzamide;
5) N-(2-(4-ethylphenylamino)benzo[d]oxazol-5-yl)-3,4-dichlorobenzamide;
6) N-(2-(4-ethylphenylamino)benzo[d]oxazol-5-yl)-3-(chloromethyl)benzamide;
7) 2-[N-(3,4-dichlorophenyl)]aminobenzoxazole;
8) N-(3,4-dichlorophenyl)naphtho [2,3 -d] oxazol-2-amine;
9) N-(3,4-difluorophenyl)-5-methylbenzo[d]oxazol-2-amine; and
10) N-(3,4-difluorophenyl)benzo[d]oxazol-2-amine.

The compound represented by Chemical Formula 1 of the present invention can be used in the form of a pharmaceutically acceptable salt, and as a salt, an acid addition salt formed by a pharmaceutically acceptable free acid is useful. The expression "pharmaceutically acceptable salt" refers to any organic or inorganic addition salt of a base compound of Chemical Formula 1 whose concentration has effective action because it is relatively non-toxic and harmless to the patients and whose side effects resulting from the salt do not degrade the beneficial efficacy of the base compound of Chemical Formula 1. These salts may use an inorganic acid and an organic acid as a free acid, as the inorganic acid, it is possible to use hydrochloric acid, bromic acid, nitric acid, sulfuric acid, perchloric acid, phosphoric acid, and the like, and as the organic acid, it is possible to use citric acid, acetic acid, lactic acid, maleic acid, fumaric acid, gluconic acid, methanesulfonic acid, glycolic acid, succinic acid, tartaric acid, galacturonic acid, embonic acid, glutamic acid, aspartic acid, oxalic acid, (D) or (L) malic acid, maleic acid, methanesulfonic acid, ethanesulfonic acid, 4-toluenesulfonic acid, salicylic acid, citric acid, benzoic acid, malonic acid, and the like. Further, these salts include alkali metal salts (sodium salts, potassium salts, and the like), alkaline earth metal salts (calcium salts, magnesium salts, and the like), and the like. For example, as an acid addition salt, acetate, aspartate, benzoate, besylate, bicarbonate/carbonate, bisulfate/sulfate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methyl sulfate, naphthylate, 2-napsylate, nicotinate, nitrate, orotate, oxalate, palmitate, pamoate, phosphate/hydrogen phosphate/dihydrogen phosphate, saccharate, stearate, succinate, tartrate, tosylate, trifluoroacetate, aluminum, arginine, benzathine, calcium, choline, diethylamine, diolamine, glycine, lysine, magnesium, meglumine, olamine, potassium, sodium, tromethamine, zinc salts, and the like may be included, and among them, hydrochloride or trifluoroacetate is preferred.

In addition, the compound represented by Chemical Formula 1 of the present invention includes not only pharmaceutically acceptable salts, but also all salts, isomers, hydrates and solvates that can be prepared by typical methods.

The addition salt according to the present invention may be prepared by a typical method, and may be prepared, for example, by dissolving the compound of Compound Formula 1 in a water-miscible organic solvent, for example, acetone, methanol, ethanol, or acetonitrile, or the like, adding an excessive amount of an organic acid thereto or adding an aqueous acid solution of an inorganic acid thereto, followed by precipitation or crystallization. Subsequently, the acid addition salt may be prepared by evaporating the solvent or excess acid from this mixture, and then drying the mixture or suction-filtering a precipitated salt.

### Preparation method 1

The present invention provides a method for preparing a compound represented by Chemical Formula 1A, as shown in the following Reaction Scheme 1, the method including:
obtaining Compound 4 by dissolving Compound 2 in an organic solvent, and then adding Compound 3 thereto, and reacting the resulting mixture at 10 to 50°C for 12 to 20 hours (Step 1); and
obtaining Compound 1A by adding an organic solvent in which Compound 4 obtained in Step 1 is dissolved dropwise to an organic solvent in which potassium superoxide is dissolved, and then reacting the resulting mixture at 15 to 30°C for 10 to 16 hours (Step 2).

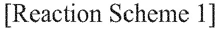

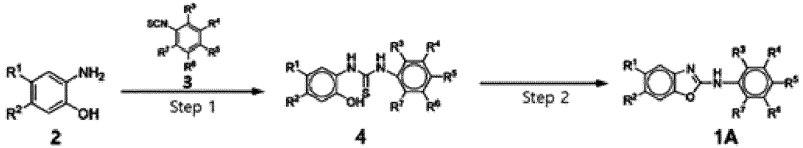

In Reaction Scheme 1,
R¹, R², R³, R⁴, R⁵, R⁶ and R⁷ are the same as defined in Chemical Formula 1 of claim 1, and
Compound 1A is included in Chemical Formula 1 of claim 1.

In the preparation method of the present invention, as an example of the organic solvent, methanol (MeOH), dimethylformamide (DMF), acetonitrile (MeCN), tetrahydrofuran (THF), dichloromethane (DCM), 1,2-dimethoxyethane, benzene, toluene, xylene, dimethyl sulfoxide (DMSO), or dioxane may be used alone or in mixtures thereof.

In the preparation method of the present invention, an example of the compound that can be prepared by the above preparation method may be 8-methyl-2-[N-(3,4-dichlorophenyl)]aminobenzoxazole, 2-[N-(3,4-dichlorophenyl)]aminobenzoxazole, N-(3,4-dichlorophenyl)naphtho[2,3-d]oxazol-2-amine, N-(3,4-difluorophenyl)-5-methylbenzo[d]oxazol-2-amine or N-(3,4-difluorophenyl)benzo[ d]oxazol-2-amine.

### Preparation method 2

The present invention provides a method for preparing a compound represented by Chemical Formula 1B, as shown in the following Reaction Scheme 2, the method including:
obtaining Compound 6 by dissolving Compound 5 in an organic solvent, and then adding Compound 3 thereto, and reacting the resulting mixture at 10 to 50°C for 12 to 20 hours (Step 1);
obtaining Compound 7 by adding an organic solvent in which Compound 6 obtained in Step 1 is dissolved dropwise to an organic solvent in which potassium superoxide is dissolved, and then reacting the resulting mixture for 12 to 24 hours (Step 2); and
obtaining Compound 1B by dissolving Compound 7 in an organic solvent, adding boron tribromide (BBn) thereto, and then reacting the resulting mixture at room temperature for 20 to 28 hours (Step 3).

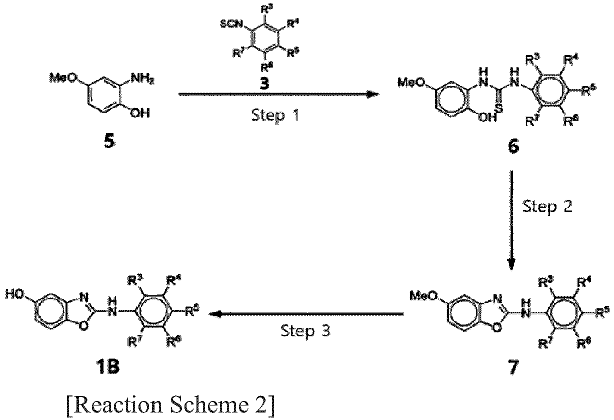

In Reaction Scheme 2,
R³, R⁴, R⁵, R⁶ and R⁷ are the same as defined in Chemical Formula 1 of claim 1, and
Compound 1B is included in Chemical Formula 1 of claim 1.

In the preparation method of the present invention, as an example of the organic solvent, methanol (MeOH), dimethylformamide (DMF), acetonitrile (MeCN), tetrahydrofuran (THF), dichloromethane (DCM), 1,2-dimethoxyethane, benzene, toluene, xylene, dimethyl sulfoxide (DMSO), or dioxane may be used alone or in combination.

In the preparation method of the present invention, an example of the compound that can be prepared by the above preparation method may be 2-((3,4-dichlorophenyl)amino)benzo[d]oxazol-5-ol.

### Preparation method 3

The present invention provides a method for preparing a compound represented by Chemical Formula 1C, as shown in the following Reaction Scheme 3, the method including:
obtaining Compound 9 by dissolving Compound 8, carbon disulfide, iodomethane and sodium hydride in an organic solvent, and then reacting the resulting solution at 10 to 50°C for 2 to 8 hours (Step 1); and
obtaining Compound 1C by dissolving Compound 9 and Compound 2 in an organic solvent, and then reacting the resulting solution for 2 to 8 hours (Step 2).

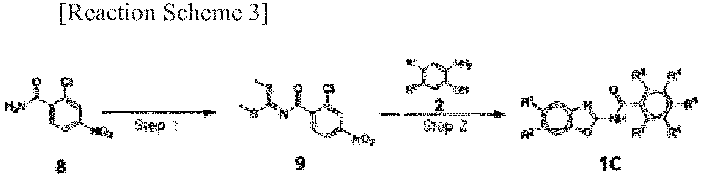

In Reaction Scheme 3,
R¹, R², R³, R⁴, R⁵, R⁶ and R⁷ are the same as defined in Chemical Formula 1 of claim 1, and
Compound 1C is included in Chemical Formula 1 of claim 1.

In the preparation method of the present invention, as an example of the organic solvent, methanol (MeOH), dimethylformamide (DMF), acetonitrile (MeCN), tetrahydrofuran (THF), dichloromethane (DCM), 1,2-dimethoxyethane, benzene, toluene, xylene, dimethyl sulfoxide (DMSO), or dioxane may be used alone or in combination.

In the preparation method of the present invention, an example of the compound that can be prepared by the above preparation method may be N-(benzo[d]oxazol-2-yl)-2-chloro-4-nitrobenzamide.

### Preparation method 4

The present invention provides a method for preparing a compound represented by Chemical Formula ID, as shown in the following Reaction Scheme 4, the method including:
obtaining Compound 11 by dissolving Compound 10 and Compound 3 in an organic solvent, and then reacting the resulting solution at 10 to 50°C for 20 to 28 hours (Step 1);
obtaining Compound 12 by adding an organic solvent in which Compound 11 obtained in Step 1 is dissolved dropwise to an organic solvent in which potassium superoxide is dissolved, and then reacting the resulting mixture at 10 to 50°C for 12 to 24 hours (Step 2);
obtaining Compound 13 by adding Compound 12 together with a catalyst to an organic solvent, and then injecting hydrogen gas thereinto, and reacting the resulting mixture at 10 to 50°C for 12 to 20 hours (Step 3); and
obtaining Compound ID by dissolving Compound 13 and Compound 14 in an organic solvent, and then reacting the resulting solution at 10 to 50°C for 12 to 24 hours (Step 4).

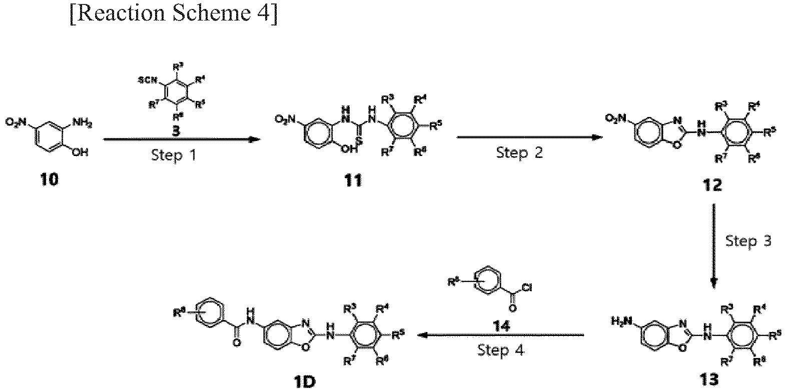

In Reaction Scheme 4,
R³, R⁴, R⁵, R⁶ and R⁷ are the same as defined in Chemical Formula 1 of claim 1;
R⁸ is one or more of a halogen, -NO₂ and a C₁₋₁₀ straight or branched alkyl halide; and
Compound ID is included in Chemical Formula 1 of claim 1.

In the preparation method of the present invention, as an example of the organic solvent, methanol (MeOH), dimethylformamide (DMF), acetonitrile (MeCN), tetrahydrofuran (THF), dichloromethane (DCM), 1,2-dimethoxyethane, benzene, toluene, xylene, dimethyl sulfoxide (DMSO), or dioxane may be used alone or in combination.

In the preparation method of the present invention, an example of the compound that can be prepared by the above preparation method may be N-(2-(4-ethylphenylamino)benzo[d]oxazol-5-yl)-2-chloro-5 -nitrobenzamide, N-(2-(4-ethylphenylamino)benzo[d]oxazol-5-yl)-3,4-dichlorobenzamide or N-(2-(4-ethylphenylamino)benzo [d]oxazol-5-yl)-3-(chloromethyl)benzamide.

### Composition for inhibiting cellular senescence

The present invention provides a composition for inhibiting cellular senescence, including the compound represented by Chemical Formula 1 or a pharmaceutically acceptable salt thereof.

In the composition of the present invention, the cells may be renal proximal tubule epithelial cells, but are not limited thereto.

In the composition of the present invention, the composition may increase the expression level of the klotho gene.

### Cosmetic composition for preventing or alleviating skin aging

The present invention provides a cosmetic composition for preventing or alleviating skin aging, including the compound represented by Chemical Formula 1 or a pharmaceutically acceptable salt thereof.

In the composition of the present invention, the composition may improve the expression level of the klotho gene.

The cosmetic composition of the present invention can be prepared by including a cosmetically effective amount of the active material of the present invention described above and a cosmetically acceptable carrier.

The cosmetic composition according to an exemplary embodiment of the present invention is not particularly limited in its formulation, and may be formulated into a cosmetic such as a toner, a skin softener, a skin toner, an astringent, a lotion, a milk lotion, a moisture lotion, a nutrition lotion, a massage cream, a nutrition cream, an eye cream, a moisture cream, a hand cream, an essence, a nutrition essence, a pack, a cleansing foam, cleansing water, a cleansing lotion, a cleansing cream, a body lotion, a body cleanser, a soap or a powder.

When the formulation of the present invention is a paste, a cream or a gel, an animal oil, a vegetable oil, a wax, paraffin, a starch, traganth, a cellulose derivative, a polyethylene glycol, silicone, bentonite, silica, talc, zinc oxide, or the like may be used as a carrier ingredient.

When the formulation of the present invention is a powder or a spray, lactose, talc, silica, aluminum hydroxide, calcium silicate, or a polyamide powder may be used as the carrier ingredient, and in particular, when the formulation of the present invention is a spray, the formulation may additionally include a propellant such as a chlorofluorohydrocarbon, propane/butane or dimethyl ether.

When the formulation of the present invention is a solution or an emulsion, a solvent, a solubilizer or an emulsifier is used as the carrier ingredient, and examples thereof include water, ethanol, isopropanol, ethyl carbonate, ethyl acetate, benzyl alcohol, benzyl benzoate, propylene glycol, 1,3-butylglycol oil, glycerol aliphatic esters, polyethylene glycol or fatty acid esters of sorbitan.

When the formulation of the present invention is a suspension, a liquid diluent such as water, ethanol or propylene glycol, a suspending agent such as ethoxylated isostearyl alcohol, polyoxyethylene sorbitol ester or polyoxyethylene sorbitan ester, microcrystalline cellulose, aluminum metahydroxide, bentonite, agar, tragacanth, or the like may be used as the carrier ingredient.

When the formulation of the present invention is a surfactant-containing cleanser, an aliphatic alcohol sulfate, an aliphatic alcohol ether sulfate, sulphosuccinic acid monoester, isethionate, an imidazolinium derivative, methyltaurate, sarcosinate, fatty acid amide ether sulfate, an alkylamidobetaine, an aliphatic alcohol, fatty acid glyceride, fatty acid diethanolamide, a vegetable oil, a lanolin derivative, an ethoxylated glycerol fatty acid ester, or the like may be used as the carrier ingredient.

### Health functional food or health food composition for preventing or alleviating skin aging

The present invention provides a health functional food or health food composition for preventing or alleviating skin aging, including the compound represented by Chemical Formula 1 or a pharmaceutically acceptable salt thereof.

In the composition of the present invention, the composition may improve the expression level of the klotho gene.

The type of food is not particularly limited. Examples of foods to which the active material of the present invention can be added include drinks, meats, sausages, bread, biscuits, rice cakes, chocolate, candies, snacks, confectioneries, pizza, instant noodles, other noodles, gums, dairy products including ice cream, various soups, drinking water, alcoholic beverages, vitamin complexes, milk products, dairy products, and the like, and include all health foods and health functional foods in a typical sense.

The health food and health functional food composition containing the active material according to the present invention may be added to food as it is or may be used together with other foods or food ingredients, and may be appropriately used according to a typical method. The mixing amount of the active material may be suitably determined depending on the purpose of use (for prevention or alleviation). In general, the amount of the composition in health foods and health functional foods may be 0.1 to 90 parts by weight of the total food weight. However, in the case of long-term intake for the purpose of maintaining health or for the purpose of health control, the amount may be equal to or less than the above range, and the effective material may be used in an amount equal to or more than the above range because it poses no problem in terms of safety.

Other ingredients are not particularly limited, other than that the health food and health functional food composition of the present invention contains the active material of the present invention as an essential ingredient at an indicated ratio, and the health food and health functional food composition of the present invention may contain various flavoring agents like those of a typical beverage, natural carbohydrates, and the like as additional ingredients. Examples of the above-described natural carbohydrates include typical sugars such as monosaccharides, for example, glucose, fructose and the like; disaccharides, for example, maltose, sucrose and the like; and polysaccharides, for example, dextrin, cyclodextrin and the like, and sugar alcohols such as xylitol, sorbitol, and erythritol. As flavoring agents in addition to those described above, a natural flavoring agent (thaumatin), a stevia extract (for example, rebaudioside A, glycyrrhizin and the like), and a synthetic flavoring agent (saccharin, aspartame and the like) may be advantageously used. The proportion of the natural carbohydrate is generally about 1 to 20 g, and preferably about 5 to 12 g per 100 g of the health functional food composition of the present invention.

The health food and health functional food composition containing the active material of the present invention may contain various nutrients, vitamins, minerals (electrolytes), flavoring agents such as synthetic flavoring agents and natural flavoring agents, colorants and thickening agents (cheese, chocolate, and the like), pectic acid and salts thereof, alginic acid and salts thereof, organic acids, protective colloid thickeners, pH adjusting agents, stabilizers, preservatives, glycerin, alcohols, carbonating agents used in carbonated beverages, or the like, in addition to the additives. In addition, the health food and health functional food composition of the present invention may contain flesh for preparing natural fruit juice, fruit juice drinks, and vegetable drinks.

These ingredients may be used either independently or in combination. The proportion of these additives is not particularly important, but is generally selected within a range of 0.1 to 20 parts by weight per 100 parts by weight of the health food and health functional food composition of the present invention containing the active material of the present invention.

### Pharmaceutical composition for preventing or treating vascular aging-induced disease

The present invention provides a pharmaceutical composition for preventing or treating a vascular aging-induced disease, including the compound represented by Chemical Formula 1 or a pharmaceutically acceptable salt thereof.

In the composition of the present invention, the composition may increase the expression level of the klotho gene.

Further, the present invention provides a pharmaceutical composition for preventing or treating a vascular aging-induced disease, including the compound represented by Chemical Formula 1 or a pharmaceutically acceptable salt thereof, and an angiotensin II receptor blocker. Specifically, the angiotensin II receptor blocker may include one or more selected from the group consisting of losartan, valsartan, telmisartan, irbesartan, azilsartan, and olmesartan, but is not limited thereto.

When the angiotensin II receptor blocker drug used as an existing therapeutic agent for hypertension is co-administered with the compound represented by Chemical Formula 1 or a pharmaceutically acceptable salt thereof, it is possible to show a better blood pressure lowering effect than when either drug is independently administered.

In the composition of the present invention, the vascular aging-induced disease may be hypertension, angina pectoris, myocardial infarction, arteriosclerosis, prostate hyperplasia, vasospasm contraction, thrombosis, cerebral infarction or cerebral hemorrhage.

The compound of the present invention may be administered in various oral and parenteral dosage forms, and during the formulation, the compound of the present invention is prepared using a diluent or an excipient, such as a filler, a thickener, a binder, a wetting agent, a disintegrant, and a surfactant which are commonly used.

A solid formulation for oral administration includes a tablet, a pill, a powder, a granule, a capsule, a troche, and the like, and the solid formulation is prepared by mixing at least one excipient, for example, a starch, calcium carbonate, sucrose or lactose, gelatin, and the like with one or more compounds of the present invention. Further, in addition to a simple excipient, lubricants such as magnesium stearate and talc are also used. A liquid preparation for oral administration corresponds to a suspension agent, a liquid for internal use, an emulsion, a syrup, and the like, and the liquid preparation may include various excipients, for example, a wetting agent, a sweetener, an aroma, a preservative, and the like, in addition to water and liquid paraffin which are commonly used simple diluents.

A preparation for parenteral administration includes an aqueous sterile solution, a non-aqueous solvent, a suspension solvent, an emulsion, a freeze-dried preparation, a suppository, or the like. As a non-aqueous solvent and a suspension solvent, it is possible to use propylene glycol, polyethylene glycol, a vegetable oil such as olive oil, an injectable ester such as ethyl oleate, and the like. As a base of the suppository, it is possible to use Witepsol, Macrogol, Tween 61, cacao butter, laurin fat, glycerol, gelatin, and the like.

In addition, the effective dosage of the compound of the present invention to the human body may vary depending on the patient's age, body weight, sex, administration form, health condition, and severity of disease, and is generally about 0.001 to 100 mg/kg/day, preferably 0.01 to 35 mg/kg/day. Based on an adult patient weighing 70 kg, the dosage is generally 0.07 to 7000 mg/day, preferably 0.7 to 2500 mg/day, and the compound of the present invention may be administered in divided doses once or several times a day at regular time intervals according to the judgment of a doctor or pharmacist.

### Health functional food or health food composition for preventing or treating vascular aging-induced disease

The present invention provides a health functional food or health food composition for preventing or treating a vascular aging-induced disease, including the compound represented by Chemical Formula 1 or a pharmaceutically acceptable salt thereof.

In the composition of the present invention, the composition may increase the expression level of the klotho gene.

In the composition of the present invention, the vascular aging-induced disease may be hypertension, angina pectoris, myocardial infarction, arteriosclerosis, prostate hyperplasia, vasospasm contraction, thrombosis, cerebral infarction or cerebral hemorrhage.

### Pharmaceutical composition for preventing or treating renal disease

The present invention provides a pharmaceutical composition for preventing or treating a renal disease, including the compound represented by Chemical Formula 1 or a pharmaceutically acceptable salt thereof.

In the composition of the present invention, the composition may increase the expression level of the klotho gene.

In the composition of the present invention, the renal disease may be nephritis, pyelitis, nephrotic syndrome, acute pyelonephritis, chronic pyelonephritis, urinary tract infection, diabetic nephropathy, chronic glomerulonephritis, acute progressive nephritis, nephrotic syndrome, microglomerular sclerosis, membranous nephropathy or membranoproliferative glomerulonephritis.

### Health functional food or health food composition for preventing or alleviating renal disease

The present invention provides a health functional food or health food composition for preventing or alleviating a renal disease, including the compound represented by Chemical Formula 1 or a pharmaceutically acceptable salt thereof.

In the composition of the present invention, the composition may increase the expression level of the klotho gene.

In the composition of the present invention, the renal disease may be nephritis, pyelitis, nephrotic syndrome, acute pyelonephritis, chronic pyelonephritis, urinary tract infection, diabetic nephropathy, chronic glomerulonephritis, acute progressive nephritis, nephrotic syndrome, microglomerular sclerosis, membranous nephropathy or membranoproliferative glomerulonephritis.

Hereinafter, the present invention will be described in more detail with reference to the following examples. However, the following examples are only for exemplifying the present invention, and the content of the present invention is not limited by the following examples.

### <Example 1> Preparation of N-(benzo[d]oxazol-2-yl)-2-chloro-4-nitrobenzamide (FCCS-17064)

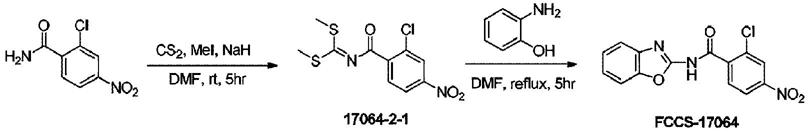

### Step 1: Preparation of dimethyl(2-chloro-4-nitrobenzoyl)carbonimidodithioate (17064-2-1)

After 2-chloro-4-nitrobenzamide (500 mg, 2.49 mmol), carbon disulfide (CS₂) (759 mg, 9.97 mmol), and iodomethane (1.13 g, 7.97 mmol) were dissolved in *N*,*N*-dimethylformamide (7 mL), 60% sodium hydride (200 mg, 4.98 mmol) was added thereto, and the resulting mixture was stirred at room temperature for 5 hours.

Ice-cold water was slowly added to the reaction mixture, and the mixture was extracted with ethyl acetate. The organic layer was washed with brine and dried over Na₂SO₄, and then the solvent was removed under reduced pressure. The reaction mixture was purified by silica-gel column chromatography (10% ethyl acetate/n-hexane) to obtain dimethyl(2-chloro-4-nitrobenzoyl)carbonimidodithioate (17064-2-1) as a light yellow solid (160 mg, 21%).

¹H NMR (400 MHz, acetone-d ₆); *δ* 8.34 (d, 1H*, J* = 2.0 Hz), 8.29 (dd, 1H*, J* = 2.4, 8.8 Hz), 8.20 (d, 1H, *J* = 8.4 Hz), 2.65 (s, 6H).

### Step 2: Preparation of N-(benzo[d]oxazol-2-yl)-2-chloro-4-nitrobenzamide (FCCS-17064)

After the dimethyl(2-chloro-4-nitrobenzoyl)carbonimidodithioate (17064-2-1) (150 mg, 0.49 mmol) obtained in Step 1 was dissolved in *N*,*N*-dimethylformamide (15 mL), 2-aminophenol (53 mg, 0.49 mmol) was added thereto.

After the reaction mixture was refluxed for 6 hours, the solvent was removed under reduced pressure. A solid obtained by adding diethyl ether to the reaction mixture and filtering a precipitated solid was purified by silica-gel column chromatography (40% ethyl acetate/n-hexane) to obtain a target compound N-(benzo[d]oxazol-2-yl)-2-chloro-4-nitrobenzamide (FCCS-17064) as a brown solid (70 mg, 30%).

¹H NMR (400 MHz, acetone-d₆); *δ* 8.36 (d, 1H, *J=* 2.0 Hz), 8.33 (dd, 1H, *J* = 2.0, 8.4 Hz), 8.09 (d, 1H, *J* = 8.4 Hz), 7.62-7.56 (m, 2H), 7.40-7.33 (m, 2H).

### <Example 2> Preparation of 8-methyl-2-[N-(3,4-dichlorophenyl)]aminobenzoxazole (FCCS-17065)

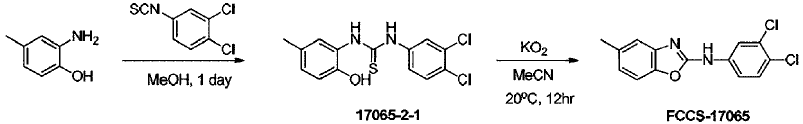

### Step 1: Preparation of 1-(3,4-dichlorophenyl)-3-(2-hydroxy-5-methylphenyl)thiourea (17065-2-1)

After 2-amino-p-cresol (300 mg, 2.44 mmol) was dissolved in methanol (12 mL), 3,4-dichlolrophenyl isothiocyanate (497 mg , 2.44 mmol) was added thereto, and the resulting mixture was stirred at room temperature for 18 hours. After the termination of the reaction was confirmed by thin layer chromatography (TLC), the resulting product was cooled in a refrigerator (0 to 4 °C). 1-(3,4-Dichlorophenyl)-3-(2-hydroxy-5-methylphenyl)thiourea (17065-2-1) as a white solid (346 mg) was obtained by filtering a precipitated solid, and was used in the next step without further purification.

¹H NMR (400 MHz, acetone-d₆); *δ* 7.98 (dd, 1H, *J* = 0.4, 2.0 Hz), 7.55-7.50 (m, 2H), 7.43 (br s, 1H), 6.94-6.90 (m, 1H), 6.85 (d, 1H, *J* = 8.4 Hz) 2.24 (s, 3H).

### Step 2: Preparation of 8-methyl-2-[N-(3,4-dichlorophenyl)laminobenzoxazole (FCCS-17065)

After the 1-(3,4-dichlorophenyl)-3-(2-hydroxy-5-methylphenyl)thiourea (17065-2-1) (346 mg, 1.06 mmol) obtained in Step 1 in a solution of potassium superoxide (KO₂) (375 mg, 5.29 mmol) and acetonitrile (MeCN) (15 mL) was slowly added to a solution dissolved in acetonitrile (MeCN) (25 mL), the resulting mixture was stirred at room temperature for 18 hours.

Dichloromethane and water were added to the reaction mixture, and the mixture was extracted. The organic layer was washed with brine and dried over Na₂SO₄, and then the solvent was removed under reduced pressure. The reaction mixture was purified by silica-gel column chromatography (10% ethyl acetate/n-hexane to obtain a target compound 8-methyl-2-[N-(3,4-dichlorophenyl)]aminobenzoxazole (FCCS-17065) as a white solid (170 mg, 24%, 2 steps).

¹H NMR (400 MHz, acetone-d₆); *δ* 8.29 (d, 1H, *J* = 2.8 Hz), 7.73 (dd, 1H, *J*= 2.8, 8.8 Hz), 7.76 (d, 1H, *J* = 8.8 Hz), 7.32-7.20 (m, 1H), 7.28 (d, 1H, J = 8.0 Hz), 7.00-6.970 (m, 1H), 2.41 (s, 3H).

### <Example 3> Preparation of 2-((3,4-dichlorophenyl)amino)benzo[d]oxazol-5-ol (FCCS-17066)

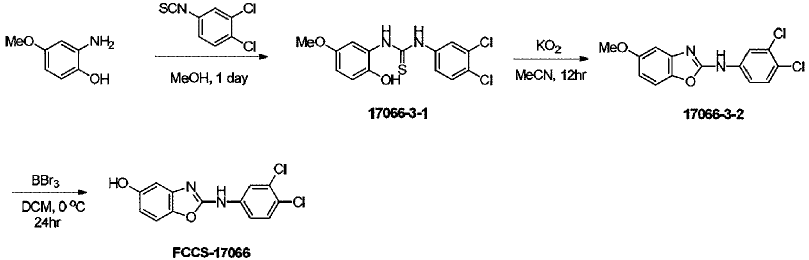

### Step 1: Preparation of 1-(3,4-dichlorophenyl)-3-(2-hydroxy-5-methoxyphenyl)thiourea (17066-3-1)

After 2-amino-4-methoxyphenol (1.13 g, 8.12 mmol) was dissolved in methanol (40 mL), 3,4-dichlolrophenyl isothiocyanate (1.99 g, 9.74 mmol) was added thereto, and the resulting mixture was stirred at room temperature for 18 hours. After the end of the reaction was confirmed by thin layer chromatography (TLC), the resulting product was cooled in a refrigerator (0 to 4 °C). 1-(3,4-Dichlorophenyl)-3-(2-hydroxy-5-methylphenyl)thiourea (17066-3-1) as a brown solid (2 g) was obtained by filtering a precipitated solid, and was used in the next step without further purification.

¹H NMR (400 MHz, methanol-d₄); *δ* 7.82 (d, 1H, *J* = 2.4 Hz), 7.48-7.44 (m, 2H), 7.39 (dd, 1H, *J* = 1.4, 8.8 Hz), 6.81 (d, 1H, *J* = 8.8 Hz), 6.66 (dd, 1H, *J* = 1.6, 8.8 Hz), 3.73 (s, 3H).

### Step 2: Preparation of N-(3,4-dichlorophenyl)-5-methoxybenzo[dloxazol-2-amine (17066-3-2)

After the 17066-3-1 (525 mg, 1.52 mmol) obtained in Step 1 in a solution of potassium superoxide (KO₂) (540 mg, 7.6 mmol) and acetonitrile (MeCN) (20 mL) was slowly added to a solution dissolved in acetonitrile (MeCN) (30 mL), the resulting mixture was stirred at room temperature for 18 hours. Dichloromethane and water were added to the reaction mixture, and the mixture was extracted. The organic layer was washed with brine and dried over Na₂SO₄, and then the solvent was removed under reduced pressure. The reaction mixture was purified by silica-gel column chromatography (20% ethyl acetate/n-hexane) to obtain N-(3,4-dichlorophenyl)-5-methoxybenzo[d]oxazol-2-amine (17066-3-2) as a brown solid (230 mg, 35%).

¹H NMR (400 MHz, acetone-d₆); δ 8.29 (d, 1H, *J* = 2.4 Hz), 7.70 (dd, 1H, *J =* 2.4, 8.8 Hz), 7.55 (d, 1H, *J* = 8.8 Hz), 7.30 (d, 1H, *J* = 8.8 Hz), 7.08 (d, 1H, *J* = 2.8 Hz), 7.74 (dd, 1H, *J* = 2.4, 8.8 Hz), 3.84 (s, 3H).

### Step 3: Preparation of 2-((3,4-dichlorophenyl)amino)benzo[d]oxazol-5-ol (FCCS-17066)

After the 17066-3-2 (200 mg, 0.65 mmol) obtained in Step 2 was dissolved in dichloromethane (15 mL, anhydrous) in an Ar gas atmosphere, the resulting solution was cooled in an ice bath. Boron tribromide (BBr₃) (3.23 mL, 1.0 M in dichloromethane) was slowly added thereto, the temperature was increased to room temperature, and then the resulting mixture was stirred for 24 hours. The reaction was terminated by slowly adding a sodium hydroxide (NaOH) solution (8 mL, 1.0 M in water) thereto and the resulting product was transferred to a separatory funnel to separate an organic layer and an aqueous layer. The aqueous layer was extracted with ethyl acetate, and then dried over Na₂SO₄, and then the solvent was removed under reduced pressure. The reaction mixture was purified by silica-gel column chromatography (40% ethyl acetate/n-hexane) to obtain a target compound 2-((3,4-dichlorophenyl)amino)benzo[d]oxazol-5-ol (FCCS-17066) as a brown solid (97 mg, 50%).

¹H NMR (400 MHz, acetone-d₆); *δ* 8.29 (br s, -OH), 8.26 (d, 1H, *J* = 2.4 Hz), 7.72 (dd, 1H, *J* = 2.4, 8.8 Hz), 7.56 (d, 1H, *J* = 8.8 Hz), 7.21 (dd, 1H, *J* = 2.0, 7.2 Hz), 6.95 (d, 1H, *J* = 2.0 Hz), 6.66 (dd, 1H, *J* = 2.4, 8.8 Hz).

### <Example 4> Preparation of N-(2-(4-ethylphenylamino)benzo[d]oxazol-5-yl)-2-chloro-5-nitrobenzamide (FCCS-17067)

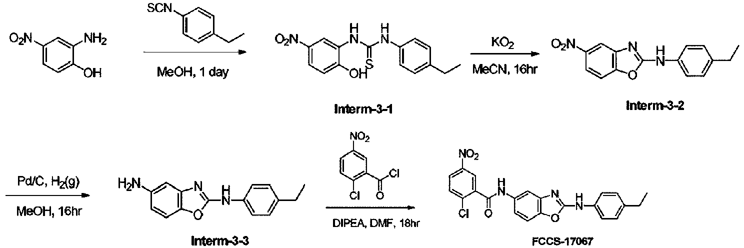

### Step 1: Preparation of 1-(4-ethylphenyl)-3-(2-hvdroxy-5-nitrophenyl)thiourea (Interm-3-1)

After 2-amino-4-nitrophenol (1.88 g, 12.25 mmol) and 4-ethylphenyl isothiocyanate (2 g, 12.25 mmol) were dissolved in methanol (80 mL), the resulting solution was stirred at room temperature overnight. After the solvent was removed under reduced pressure, the residue was purified by silica-gel column chromatography (20% ethyl acetate/n-hexane) to obtain 1-(4-ethylphenyl)-3-(2-hydroxy-5-nitrophenyl)thiourea (Interm-3-1) as a brown solid (2.9 g, 65%).

¹H NMR (400 MHz, methanol-d₄); δ 9.24 (d, 1H, *J* = 2.8 Hz), 7.88 (dd, 1H, *J* = 2.0, 9.2 Hz), 7.36 (d, 2H, *J=* 8.4 Hz), 7.25 (d, 2H, *J=* 8.8 Hz), 6.94 (d, 1H, *J=* 9.2 Hz), 2.66 (q, 2H, *J* = 7.6 Hz), 1.24 (t, 3H, *J* = 7.6 Hz).

### Step 2: Preparation of N-(4-ethylphenyl)-5-nitrobenzo[d]oxazol-2-amine (Interm-3-2)

After a solution of potassium superoxide (KO₂) (2.8 g, 39.38 mmol) and acetonitrile (MeCN) (130 mL) was cooled in an ice bath, a solution of the Interm-3-1 (2.5 g, 7.88 mmol) obtained in Step 1 dissolved in acetonitrile (MeCN) (170 mL) was slowly added thereto, and then the resulting solution was stirred at room temperature for 18 hours. Dichloromethane and water were added to the reaction mixture, and the mixture was extracted. The organic layer was washed with brine and dried over Na₂SO₄, and then the solvent was removed under reduced pressure. The reaction mixture was purified by silica-gel column chromatography (10% ethyl acetate/n-hexane) to obtain Compound Interm-3-2 as a brown solid (1.78 g, 80%).

¹H NMR (400 MHz, methanol-d₄); *δ* 8.20 (d, 1H, *J=* 1.0 Hz), 8.08 (dd, 1H, *J* = 0.8, 9.6 Hz), 7.59 (d, 2H, J= 8.8 Hz), 7.51 (d, 1H, *J=* 8.8 Hz), 7.22 (d, 2H, *J=* 8.8 Hz), 2.64 (q, 2H, *J* = 7.6 Hz), 1.24 (t, 3H, *J=* 7.6 Hz).

### Step 3: Preparation of N-(4-ethylphenyl)benzo[d]oxazole-2,5-diamine (Interm-3-3)

After palladium on carbon (Pd/C) (1.70 g, 0.80 mmol, 10 wt%, wet support) was weighed and put into a round-bottom flask, the flask was purged with Ar gas. After a solution of the Interm-3-2 (1.58 g, 5.30 mmol) obtained in Step 2 dissolved in methanol (80 mL) was slowly added thereto, the atmosphere in the flask was substituted with H₂(g). The solution was stirred at room temperature for 18 hours while bubbling H₂(g). After the termination of the reaction was confirmed by thin layer chromatography (TLC), the resulting product was filtered with a Celite pad, and then the solvent was removed under reduced pressure. The reaction mixture was purified by silica-gel column chromatography (40% ethyl acetate/n-hexane) to obtain Compound Interm-3-3 as a light brown solid (1.21 g, 90%).

¹H NMR (400 MHz, Acetone-d₆); δ 7.71 (m, 2 H), 7.19 (m, 2H), 7.03 (dd, 1H, *J=* 0.8, 8.4 Hz), 6.75 (dd, 1H, *J=* 0.8, 2.0 Hz), 6.44 (dd, 1H, *J=* 2.0, 8.4 Hz), 2.60 (q, 2H*, J* = 7.6 Hz), 1.19 (t, 3H, *J* = 7.6 Hz).

### Step 4: Preparation of N-(2-(4-ethylphenylamino)benzo[d]oxazol-5-yl)-2-chloro-5-nitrobenzamide (FCCS-17067)

After the Interm-3-3 (253 mg, 1 mmol) obtained in Step 3 and 2-chloro-5-nitrobenzoyl chloride (220 mg, 1 mmol) were dissolved in *N*,*N*-dimethylformamide (DMF) (4 mL), diisopropylethylamine (DIPEA) (129 mg, 1 mmol) was added thereto, and the resulting mixture was stirred at room temperature for 18 hours. After 18 hours, 0.5 equiv. of each of 2-chloro-5-nitrobenzoyl chloride and diisopropylethylamine (DIPEA) was added thereto, and the resulting mixture was further stirred for 8 hours. 10% HCl (aq.) was added to the reaction mixture, the mixture was extracted with ethyl acetate, and then the organic layer was sequentially washed with a saturated aqueous NaHCO₃ solution and brine. After the organic layer was dried over Na₂SO₄, the solvent was removed under reduced pressure. The reaction mixture was purified by silica-gel column chromatography (40% ethyl acetate/n-hexane) to obtain a target compound N-(2-(4-ethylphenylamino)benzo[d]oxazol-5-yl)-2-chloro-5-nitrobenzamide (FCCS-17067) as a light yellow solid (120 mg, 27%).

¹H NMR (400 MHz, DMSO-d₆); *δ* 10.71 (s, 1H), 10.53 (s, 1H), 8.48 (d, 1H, *J* = 2.8 Hz), 8.34 (dd, 1H, *J* = 2.4, 8.8 Hz), 7.90 (d, 1H, *J* = 8.8 Hz), 7.82 (d, 1H, *J* = 2.0 Hz), 7.64 (d, 2H, *J* = 8.8 Hz), 7.46 (d, 1H, *J* = 8.8 Hz), 7.40 (dd, 1H, *J* = 2.0, 8.4 Hz), 7.21 (d, 1H, *J* = 8.8 Hz), 2.58 (q, 2H, *J* = 7.6 Hz), 1.18 (t, 3H, *J* = 7.6 Hz).

### <Example 5> Preparation of N-(2-(4-ethylphenylamino)benzo[d]oxazol-5-yl)-3,4-dichlorobenzamide (FCCS-17068)

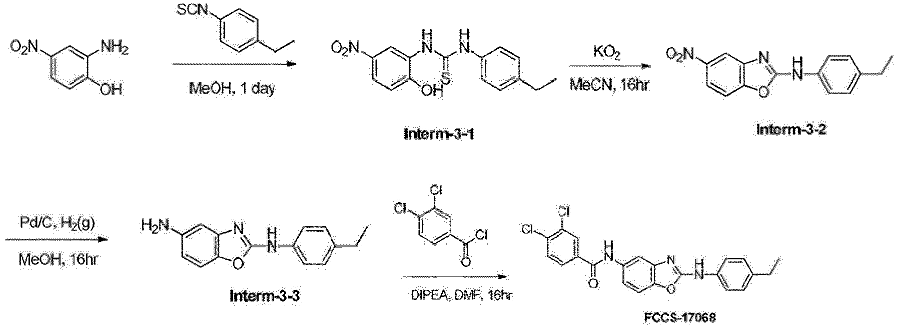

After the Interm-3-3 (253 mg, 1 mmol) obtained in Step 3 in Example 4 and 3,4-dichlorobenzoyl chloride (209 mg, 1 mmol) were dissolved in *N,N-*dimethylformamide (DMF) (4 mL), diisopropylethylamine (DIPEA) (129 mg, 1 mmol) was added thereto, and the resulting mixture was stirred at room temperature for 18 hours. 10% HCl (aq.) was added to the reaction mixture, the mixture was extracted with ethyl acetate, and then the organic layer was sequentially washed with a saturated aqueous NaHCO₃ solution and brine. After the organic layer was dried over Na₂SO₄, the solvent was removed under reduced pressure. The reaction mixture was purified by silica-gel column chromatography (40% ethyl acetate/n-hexane) to obtain a target compound FCCS-17067 as a light white solid (270 mg, 64%).

¹H NMR (400 MHz, Acetone-d₆); *δ* 8.18 (d, 1H, *J* = 2.4 Hz), 7.99 (t, 1H, *J*= 2.4 Hz), 7.97 (d, 1H, *J=* 2.0 Hz), 7.80-7.20 (m, 3H), 7.70-7.50 (m, 1H), 7.35 (d, 1H, *J* = 8.8 Hz), 7.24 (d, 1H, *J* = 8.8 Hz), 2.63 (q, 2H, *J* = 7.6 Hz), 1.22 (t, 3H, *J* = 7.6 Hz).

### <Example 6> Preparation of N-(2-(4-ethylphenylamino)benzo[d]oxazol-5-yl)-3-(chloromethyl)benzamide (FCCS-17069)

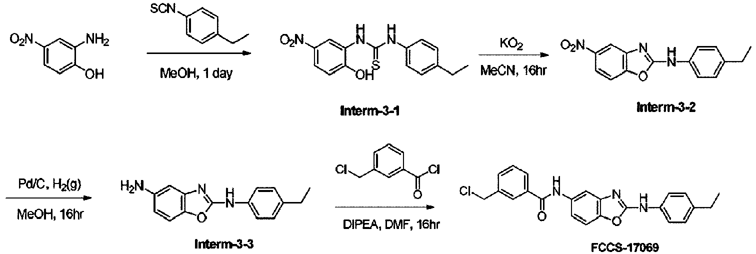

After the Interm-3-3 (253 mg, 1 mmol) obtained in Step 3 in Example 4 and 3-(chloromethyl)benzoyl chloride (189 mg, 1 mmol) were dissolved in *N,N-*dimethylformamide (DMF) (4 mL), diisopropylethylamine (DIPEA) (129 mg, 1 mmol) was added thereto, and the resulting mixture was stirred at room temperature for 18 hours. 10% HCl (aq.) was added to the reaction mixture, the mixture was extracted with ethyl acetate, and then the organic layer was sequentially washed with a saturated aqueous solution of NaHCO₃ and brine. After the organic layer was dried over Na₂SO₄, the solvent was removed under reduced pressure. The reaction mixture was purified by silica-gel column chromatography (30% ethyl acetate/n-hexane) to obtain a target compound FCCS-17067 as a pale white solid (170 mg, 40 %).

¹H NMR (400 MHz, Acetone-d₆); *δ* 8.08 (t, 1H, *J* = 1.2 Hz), 8.03 (d, 1H, *J*= 2.0 Hz), 7.98 (dt, 1H, *J* = 1.2, 7.6 Hz), 7.80-7.75 (m, 2H), 7.69-7.65 (m, 1H), 7.57-7.52 (m, 3H), 7.34 (d, 1H, *J* = 8.8 Hz), 7.26-7.22 (m, 2H), 2.62 (q, 2H, *J* = 7.6 Hz), 1.22 (t, 3H, *J=* 7.6 Hz).

### <Example 7> Preparation of 2-[N-(3,4-dichlorophenyl)]aminobenzoxazole (FCCS-17065-A)

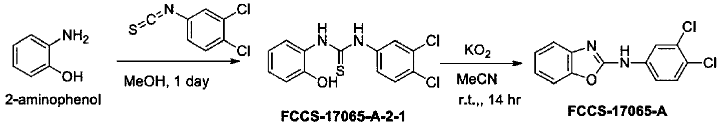

### Step 1: Preparation of 1-(3,4-dichlorophenyl)-3-(2-hydroxyphenyl)thiourea (FCCS-17065-A-2-1)

After methanol (anhydrous MeOH) (8 mL) was added to and dissolved in 2-aminophenol (300 mg, 2.749 mmol) in an Ar gas atmosphere, 3,4-dichlorophenyl isothiocyanate (0.47 mL, 3.299 mmol) was slowly added dropwise thereto and the resulting mixture was stirred at room temperature for 14 hours. It was confirmed by thin layer chromatography (TLC) that all starting materials had disappeared, the solvent was removed by reducing pressure, and then silica was added to and adsorbed to a crude product, and silica-gel flash column chromatography (30% EtOAc/hexane, R_{f}=0.4) was performed to obtain 793 mg (light brown foamy solid, 92%) of 1-(3,4-dichlorophenyl)-3-(2-hydroxyphenyl)thiourea (FCCS-17065-A-2-1).

¹H NMR (400 MHz, CD3OD); *δ* 7.82 (d, 1 H, *J* = 2.8 Hz), 7.63 (d, 1 H, J = 7.6 Hz), 7.45 (d, 1 H, *J* = 8.8 Hz), 7.39 (dd, 1 H, *J* = 8.6, 2.2 Hz), 7.11-7.06 (m, 1 H), 6.90 (dd, 1 *H, J* = 8.0, 1.2 Hz), 6.85 (td, 1 H, *J* = 7.6, 1.2 Hz)

### Step 2: Preparation of 2-[N-(3,4-dichlorophenyl)laminobenzoxazole (FCCS-17065-A)

The FCCS-17065-A-2-1 (400 mg, 1.277 mmol) obtained in Step 1 and potassium superoxide (KO₂) (454 mg, 6.386 mmol) were put into a container in an Ar gas atmosphere, acetonitrile (MeCN) (48 mL) was added thereto, and the resulting mixture was stirred at room temperature for 14 hours. After it was confirmed by thin layer chromatography (TLC) that all starting materials had disappeared, silica was added to and adsorbed to a crude product crude, and pressure was reduced. Silica-gel flash column chromatography (20% EtOAc/hexane, R_{f}=0.4) was performed to obtain 231 mg (white solid, 65%) of a target compound 2-[N-(3,4-dichlorophenyl)]aminobenzoxazole (FCCS-17065-A).

¹H NMR (400 MHz, CD₃OD); *δ* 8.06 (d, 1 H, *J* = 2.8 Hz), 7.55 (dd, 1 H, *J* = 8.6, 2,6 Hz), 7.48-7.45 (m, 2 H), 7.39 (d, 1 H, *J* = 8.0 Hz), 7.24 (td, 1 H, *J* = 7.6, 1.2 Hz), 7.16 (td, 1 H, *J* = 7.8, 1.2 Hz)

### <Example 8> Preparation of N-(3,4-dichlorophenyl)naphtho[2,3-d]oxazol-2-amine (FCCS-17065-B)

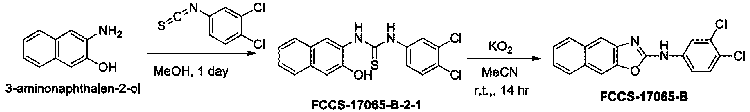

### Step 1: Preparation of 1-(3,4-dichlorophenyl)-3-(3-hydroxynaphthalen-2-yl)thiourea (FCCS-17065-B-2-1)

After methanol (anhydrous MeOH) (7 mL) and chloroform (CHCb) (2 mL) were added to 3-amino-2-naphthol (350 mg, 2.119 mmol) in an Ar gas atmosphere and the resulting mixture was stirred at room temperature for 5 minutes, 3,4-dichlorophenyl isothiocyanate (0.38 mL, 2.638 mmol) was slowly added dropwise thereto and the resulting mixture was stirred at room temperature for 13 hours. After it was confirmed by thin layer chromatography (TLC) that all starting materials had disappeared and the solvent was removed by reducing pressure, dichloromethane (8 mL) was added thereto, the resulting mixture was stirred for 5 minutes, and then a solid which had not been dissolved was filtered to obtain 792 mg (white solid, 99%) of 1-(3,4-dichlorophenyl)-3-(3-hydroxynaphthalen-2-yl)thiourea (FCCS-17065-B-2-1).

¹H NMR (400 MHz, DMSO-d₆); *δ* 10.48 (s, 1 H), 10.34 (s, 1 H), 9.57 (s, 1 H), 8.59 (s, 1 H), 8.05 (d, 1 H, *J* = 2.0 Hz), 7.73 (d, 1 H, *J* = 8.0 Hz), 7.67 (d, 1 H, *J* = 7.6 Hz), 7.60 (d, 1 H, *J* = 8.4 Hz), 7.52 (dd, 1 H, *J* = 8.6, 2.2 Hz), 7.35 (t, 1 H, *J* = 7.2 Hz), 7.27 (t, 1 H, *J* = 7.6 Hz), 7.24 (s, 1 H)

### Step 2: Preparation of N-(3,4-dichlorophenyl)naphtho[2,3-d]oxazol-2-amine (FCCS-17065-B)

The FCCS-17065-B-2-1 (400 mg, 1.101 mmol) obtained in Step 1 and potassium superoxide (KO₂) (391 mg, 5.505 mmol) were put into a container in an Ar gas atmosphere, acetonitrile (MeCN) (42 mL) was added thereto, and the resulting mixture was stirred at room temperature for 14 hours. After it was confirmed by thin layer chromatography (TLC) that all starting materials had disappeared, silica was added to and adsorbed to a crude product crude, and pressure was reduced. Silica-gel flash column chromatography (20% EtOAc/hexane, R_{f}=0.5) was performed to obtain 236 mg (white solid, 65%) of a target compound N-(3,4-dichlorophenyl)naphtho[2,3-d]oxazol-2-amine (FCCS-17065-B).

¹H NMR (400 MHz, DMSO-d6); *δ* 11.22 (s, 1 H), 8.20 (d, 1 H, *J* = 2.0 Hz), 7.98-7.95 (m, 4 H), 7.72 (dd, 1 H, *J* = 8.8, 2.4 Hz), 7.66 (d, 1 H, *J* = 8.4 Hz), 7.47-7.41 (m, 2 H)

### <Example 9> Preparation of N-(3,4-difluorophenyl)-5-methylbenzo[d]oxazol-2-amine (FCCS-17065-C)

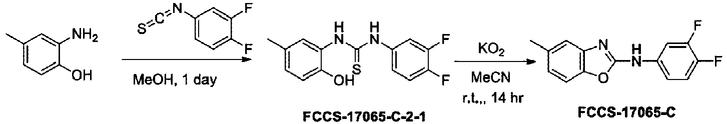

### Step 1: Preparation of 1-(3,4-difluorophenyl)-3-(2-hydroxy-5-methylphenyl)thiourea(FCCS-17065-C-2-1)

After methanol (anhydrous MeOH) (8 mL) was added to and dissolved in 2-amino-p-cresol (300 mg, 2.436 mmol) in an Ar gas atmosphere, 3,4-difluorophenyl isothiocyanate (0.37 mL, 2.923 mmol) was slowly added dropwise thereto and the resulting mixture was stirred at room temperature for 13 hours. It was confirmed by thin layer chromatography (TLC) that all starting materials had disappeared, the solvent was removed by reducing pressure, and then silica was added to and adsorbed to a crude product, and silica-gel flash column chromatography (30% EtOAc/hexane, R_{f}=0.4) was performed to obtain 710 mg (white foamy solid, 99%) of 1-(3,4-difluorophenyl)-3-(2-hydroxy-5-methylphenyl)thiourea (FCCS-17065-C-2-1).

¹H NMR (400 MHz, CD₃OD); *δ* 7.57-7.52 (m, 1 H), 7.40 (s, 1 H), 7.25-7.13 (m, 2 H), 6.91 (dd, 1 H, *J* = 8.0, 1.6 Hz), 6.79 (d, 1 H, *J* = 8.0 Hz), 2.25 (s, 3 H)

### Step 2: Preparation of N-(3,4-difluorophenyl)-5-methylbenzo[d]oxazol-2-amine (FCCS-17065-C)

The FCCS-17065-C-2-1 (400 mg, 1.359 mmol) obtained in Step 1 and potassium superoxide (KO₂) (483 mg, 6.795 mmol) were put into a container in an Ar gas atmosphere, acetonitrile (MeCN) (52 mL) was added thereto, and the resulting mixture was stirred at room temperature for 14 hours. After it was confirmed by thin layer chromatography (TLC) that all starting materials had disappeared, silica was added to and adsorbed to a crude product crude, and pressure was reduced. Silica-gel flash column chromatography (20% EtOAc/hexane, R_{f}=0.45) was performed to obtain 224 mg (white solid, 63%) of a target compound N-(3,4-difluorophenyl)-5-methylbenzo[d]oxazol-2-amine (FCCS-17065-C).

¹H NMR (400 MHz, CD₃OD); δ = 7.83-7.78 (m, 1 H), 7.33-7.29 (m, 1 H), 7.27-7.20 (m, 3 H), 6.97-6.95 (m, 1 H), 2.41 (s, 3 H)

### <Example 10> Synthesis of N-(3,4-difluorophenyl)benzo[d]oxazol-2-amine (FCCS-19025)

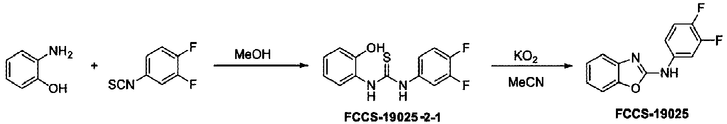

### Step 1: Preparation of 1-(3,4-difluorophenyl)-3-(2-hydroxyphenyl)thiourea (FCCS-19025-2-1)

After 2-aminophenol (150 mg, 1.37 mmol) was dissolved in methanol (8 mL) in an Ar gas atmosphere, 3,4-difluorophenyl isothiocyanate (224 µl, 1.65 mmol) was slowly added thereto, and then the resulting mixture was stirred at room temperature for 13 hours. After the termination of the reaction was confirmed by thin layer chromatography (TLC), methanol was removed under reduced pressure. The reaction mixture was purified by silica-gel column chromatography (20% acetone/n-hexane) to obtain 1-(3,4-difluorophenyl)-3-(2-hydroxyphenyl)thiourea (FCCS-19025-2-1) as a light yellow solid (354 mg, 92%).

¹H-NMR (400 MHz, MeOH-d4) *δ* 7.62 (d, *J* = 8.0 Hz, 1H), 7.55 (ddd, *J=* 2.4 Hz, 1H), 7.25-7.13 (m, 2H), 7.11-7.05 (m, 1H), 6.92-6.82 (m, 2H); ESI-(+) 281.3 [M+H]⁺.

### Step 2: Preparation of N-(3,4-difhiorophenyl)benzo[d]oxazol-2-amine (FCCS-19025)

The FCCS-19025-2-1 (224 mg, 0.80 mmol) obtained in Step 1 and potassium superoxide (KO₂) (284 mg, 4.00 mmol) were dissolved in acetonitrile (MeCN) (25 mL) in an Ar gas atmosphere, the resulting mixture was stirred at room temperature for 14 hours. After the termination of the reaction was confirmed by thin layer chromatography (TLC), acetonitrile (MeCN) was removed under reduced pressure. The reaction mixture was purified by silica-gel column chromatography (10 to 20% ethyl acetate/n-hexane) to obtain a target compound N-(3,4-difluorophenyl)benzo[d]oxazol-2-amine (FCCS-19025) as a white solid (160 mg, 82%).

¹H-NMR (400 MHz, MeOH-d4) *δ* 7.82 (ddd, J= 2.8 Hz, 1H), 7.42 (d, *J* = 7.6 Hz, 1H), 7.36 (d, *J* = 8.0 Hz, 1H), 7.34-7.29 (m, 1H), 7.28-7.18 (m, 2H), 7.16-7.10 (m, 1H); ESI-(+) 247.2 [M+H]⁺.

The chemical structural formulae of Examples 1 to 10 are shown in the following Table 1.

**[Table 1]**

| Example | Chemical structural formula | Example | Chemical structural formula |
|---|---|---|---|
| Example 1 (FCCS-17064) | 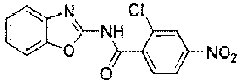 | Example 6 (FC CS-17069) | 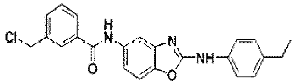 |
| Example 2 (FCCS-17065) | 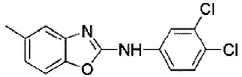 | Example 7 (FCCS-17065-A) | 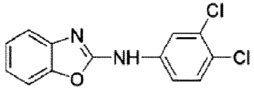 |
| Example 3 (FCCS-17066) | 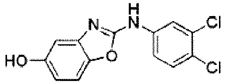 | Example 8 (FCCS-17065-B) | 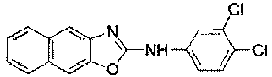 |
| Example 4 (FCCS-17067) | 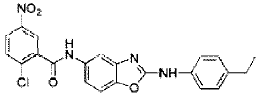 | Example 9 (FCCS-17065-C) | 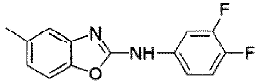 |
| Example 5 (FCCS-17068) | 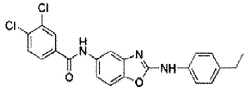 | Example 10 (FCCS-19025) | 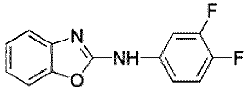 |

### <Comparative Example 1>

N-(2-chlorophenyl)-1H-indole-3-carboxamide was used as Comparative Example 1.

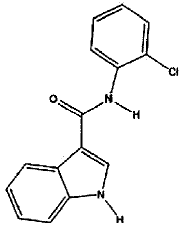

### <Comparative Example 2>

2'-Chloroacetanilide (C0621) was purchased and used as Comparative Example 2.

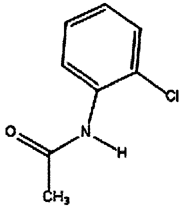

### <Comparative Example 3>

N-methyl-1H-indole-3-carboxamide (FCCS-16030) was purchased and used as Comparative Example 3.

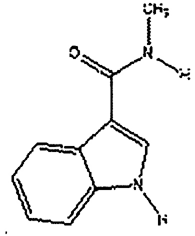

### <Comparative Example 4> Preparation of N-(2-((2-chlorophenyl)amino)-2-oxoethyl)-1H-indole-3-carboxamide (FCCS-16031)

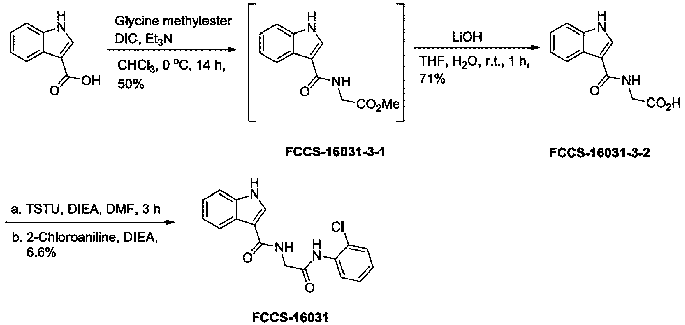

### Step 1: Preparation of methyl 2-(1H-indole-3-carboxamido)acetate (CCS-16031-3-1)

Indole-3-carboxylic acid (600 mg, 3.72 mmol) and glycine methyl ester (467 mg, 3.72 mmol) were dissolved in chloroform (11 mL), and the resulting solution was cooled in an ice bath. After triethylamine (1.04 mL, 7.446 mmol) and N,N-diisopropylcarbodiimide were additionally added thereto, the resulting mixture was stirred at 0°C for 14 hours. The mixture was washed with a 10% aqueous NaHCO₃ solution, and then washed with a 5% HCl aqueous solution, and the remaining moisture was removed by allowing the resulting mixture to pass through an anhydrous Na₂SO₄ pad, and then the solvent was removed under reduced pressure. 430 mg (white solid, 50%) of methyl 2-(1H-indole-3-carboxamido)acetate (CCS-16031-3-1) was obtained in a mixture state by performing silica-gel flash column chromatography (70% ethyl acetate/n-hexane). The next step was performed without further purification. ESI-MS : 231.2 [M-H]⁻

### Step 2: Preparation of 2-(1H-indole-3-carboxamido)acetic acid (FCCS-16031-3-2)

The methyl 2-(1H-indole-3-carboxamido)acetate (CCS-16031-3-1) (220 mg, 0.947 mmol) was dissolved in tetrahydrofuran (6 mL), a solution of a lithium hydroxide hydrate (LiOH monohydrate) (131 mg, 3.126 mmol) dissolved in water (2 mL) was added thereto, and the resulting mixture was stirred for 1 hour. After the pH was adjusted to 2 by adding a 1.0 N aqueous HCl solution, the mixture was extracted with ethyl acetate. After the remaining moisture was removed by allowing the mixture to pass through an anhydrous Na₂SO₄ pad, the solvent was removed under reduced pressure. 147 mg (yellow foamy solid, 71%) of 2-(1H-indole-3-carboxamido)acetic acid (FCCS-16031-3-2) was obtained by performing silica-gel flash column chromatography (10% methanol/dichloromethane).

¹H NMR (400 MHz, CD₃OD); δ 8.10-8.08 (m, 1 H), 7.92 (s, 1 H), 7.43 (dt, *J* = 8.0, 1.2 Hz, 1 H), 7.17 (quint d, *J* = 7.2, 1.6 Hz, 2 H), 4.12 (s, 2 H)

### Step 3: Preparation of N-(2-((2-chlorophenyl)amino)-2-oxoethyl)-1H-indole-3-carboxamide (FCCS-16031)

The 2-(1H-indole-3-carboxamido)acetic acid (FCCS-16031-3-2) (200 mg, 0.917 mmol) and N,N,N',N'-tetramethyl-O-(N-succinimidyl)uronium tetrafluoroborate (TSTU) (290 mg, 0.962 mmol) were dissolved in N,N-dimethylformamide (4 mL, anhydrous) in an Ar gas atmosphere, N,N-diisopropylethylamine (DIEA) (0.4 mL, 2.293 mmol) was added thereto, and then the resulting mixture was stirred at room temperature for 3 hours. 2-Chloroaniline (0.29 mL, 2.751 mmol) and N,N-diisopropylethylamine (DIEA) (0.64 mL, 3.668 mmol) were added thereto, and the resulting mixture was heated at 60°C for 4 hours. After the solvent was removed under reduced pressure, an organic layer was obtained by adding dichloromethane and a saturated aqueous NH₄Cl solution thereto for extraction, the remaining moisture was removed by allowing the organic layer to pass through an anhydrous Na₂SO₄ pad, and then the solvent was removed under reduced pressure. 20 mg (white solid, 6.6%) of a target compound N-(2-((2-chlorophenyl)amino)-2-oxoethyl)-1H-indole-3-carboxamide (FCCS-16031) was obtained by performing silica-gel flash column chromatography (70% ethyl acetate/n-hexane).

1H NMR (400 MHz, CD₃OD); *δ* 8.15-8.12 (m, 1 H), 8.04 (dd, *J* = 8.0, 1.2 Hz, 1 H), 7.97 (s, 1 H), 7.46-7.41 (m, 2 H), 7.33-7.28 (m, 1 H), 7.23-7.12 (m, 3 H), 4.26 (s, 2 H)

### <Experimental Example 1-1> Luciferase expression experiment (Comparative Examples 1 to 4)

In order to evaluate whether the klotho gene was expressed by the evaluation of luciferase activity, human renal proximal tubule epithelial cells (RPTECs) (ATCC CRL-4031), which are epithelial cells of the proximal tubule of the human kidneys, were purchased from Lonza, USA and used.

For culturing, a Renal Epithelial Growth Medium (REGM^{™}) Bullet kit, also manufactured by the same Lonza company, was used, and the cells were cultured under conditions of 37 °C and 5% CO₂. As a plasmid for luciferase expression, a plasmid in which the promoter site of a human KL (klotho) gene was disposed to regulate the expression of a firefly luciferase gene was used.

The plasmid was incorporated into cells using an X-treme GENE transfection reagent from Roche. The activity of luciferase expressed in cells was measured using a Dual-Luciferase reporter assay system manufactured by Promega. After the cultured cells were treated with each compound at an indicated concentration for 24 hours, the activity of luciferase was measured. It is indirectly shown that the expression of the klotho gene may be increased when the activity of luciferase is high.

The expression of a reporter gene was confirmed by treating RPTEC cells with Comparative Examples 1 to 4 at a concentration of 5 µM and using a reporter gene including a promoter from the start site of the human klotho gene to a front of 1.7 kbp or a reporter gene including a promoter from the start site of the human klotho gene to a front of 240 bp.

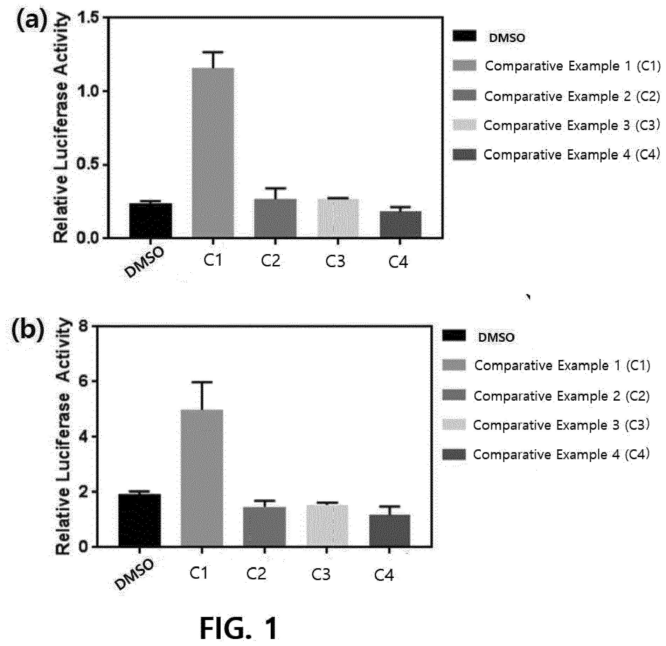

As a result, as illustrated in FIG. 1, it was confirmed that the luciferase activity of the compound of Comparative Example 1 was the highest.

### <Experimental Example 1-2> Luciferase expression experiment (Examples 1 to 6)

Based on the results of Experimental Example 1-1, Examples 1 to 6 having a chemical structure similar to that of Comparative Example 1 were synthesized, and the present Experimental Example 1-2 was performed.

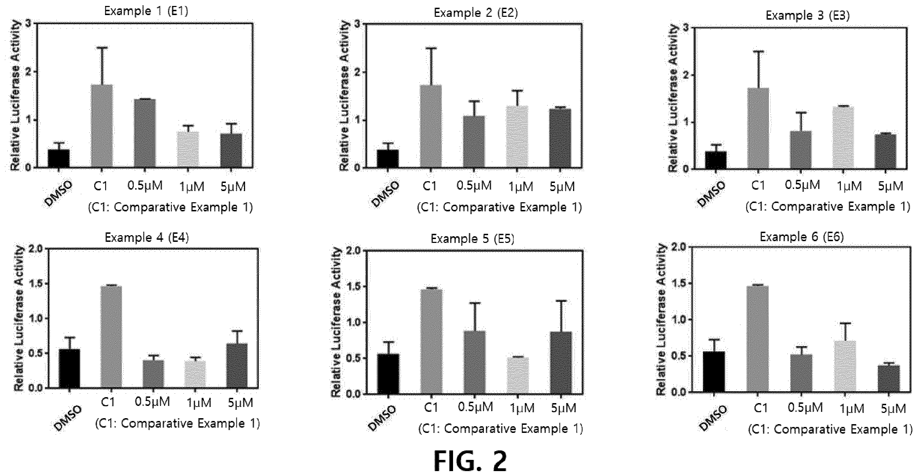

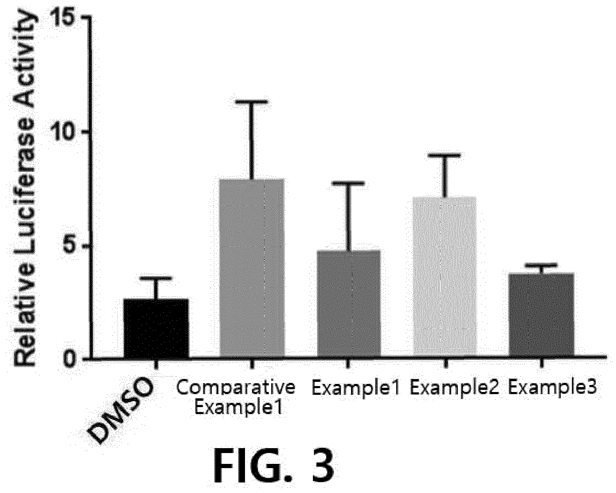

The results of confirming the expression of a reporter gene by treating RPTECs, which are epithelial cells of the proximal tubule of the human kidneys, with Examples 1 to 6 at concentrations of 0.5, 1 and 5 µM and Comparative Example 1 at a concentration of 5 µM and using a reporter gene (pHKP-luc) including a promoter included up to -2.1 kb upstream of the human klotho gene are illustrated in FIGS. 2 and 3.

As illustrated in FIG. 2, it was confirmed that the expression of the reporter genes of the compounds of Examples 1 and 2 was at a level similar to that of Comparative Example 1.

As illustrated in FIG. 3, as a result of confirming the expression of a reporter gene by treating RPTEC cells with Comparative Example 1 and Examples 1 to 3 at a concentration of 5 µM and using the reporter gene (pHKP-luc) including a promoter included up to -2.1 kb upstream of the human klotho gene, it was confirmed that the compound of Example 2 was at a level similar to that of Comparative Example 1.

### <Experimental Example 2> Quantitative evaluation of klotho (KL) gene expression level using real-time PCR experiment

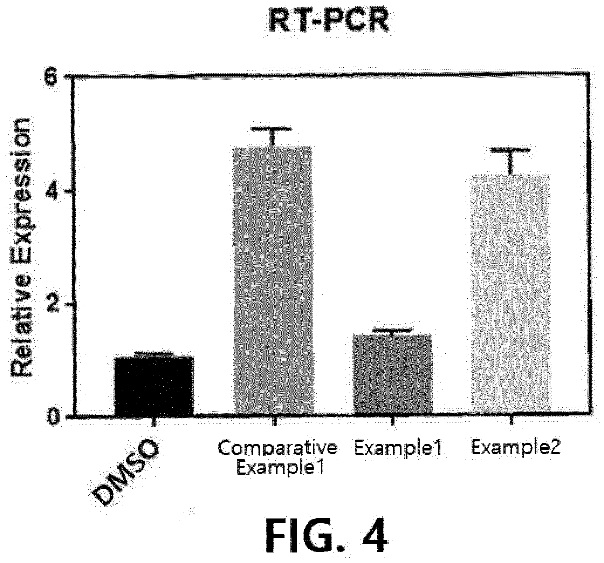

For RNA extraction from RPTEC cells, which are epithelial cells of the proximal tubule of the human kidneys, treated with the compounds of Comparative Example 1 and Examples 1 and 2 for 6 hours, an RNeasy kit from Qiagen Inc. was used. The results of preparing cDNA from extracted RNA using a Superscript II kit from Thermo Fisher Scientific, Inc. and performing the quantitative evaluation using Taqman Gene Expression assays from Applied Biosystems as a klotho (KL) gene-specific kit are illustrated in FIG. 4.

As illustrated in FIG. 4, it was confirmed that the compound of Example 2 was at a level similar to that of Comparative Example 1.

Based on the results of the present Experimental Example 2, the compounds of Examples 7 to 10 having a chemical structure similar to the compound of Example 2 were synthesized and used in the following Experimental Example 3.

### <Experimental Example 3> Quantitative evaluation of klotho (KL) gene expression level using general PCR experiment

After RPTEC cells, which are epithelial cells of the proximal tubule of the human kidneys, were each treated with the compounds of Examples 7 to 10 at 2.5 µM for 6 hours, RNA was extracted from the cells, and cDNA was prepared from the extracted RNA using a Superscript II kit from Thermo Fisher Scientific, Inc., and then general PCR was performed.

Information on the primers used in the experiment is as follows.
KL-F GATAGAGAAAAATGGCTTCCCTCC (SEQ ID NO: 1)
KL-R GGTCGGTAAACTGAGACAGAGTGG (SEQ ID NO: 2)
GAPDH-F TGACAACTTTGGTATCGTGGAAGG (SEQ ID NO: 3)
GAPDH-R AGGGATGATGTTCTGGAGAGCC (SEQ ID NO: 4)

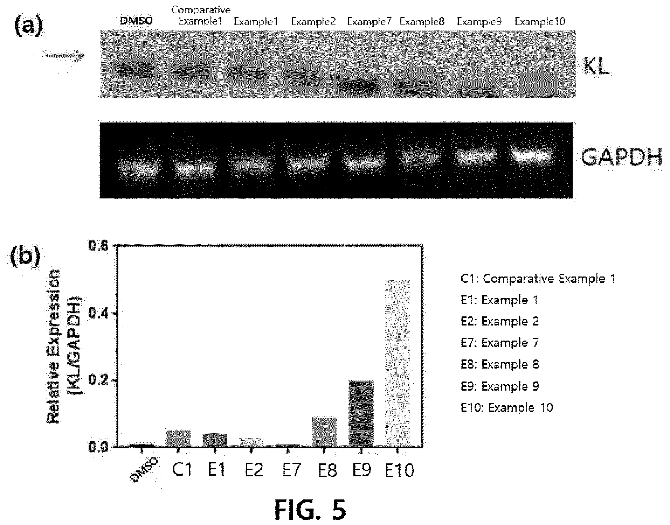

The DNA amplified by PCR was confirmed by staining with ethidium bromide after electrophoresis on an agarose gel, and the amounts of DNA in the band were quantitatively compared using the SPEedyQuant program, and are illustrated in FIG. 5.

As illustrated in FIG. 5, it was confirmed that the expression level of the klotho (KL) gene in Examples 8 to 10 was much higher than that in Comparative Example 1, and in particular, Example 10 showed an improvement to about 10 times the level of Comparative Example 1.

### <Experimental Example 4> Toxicity test

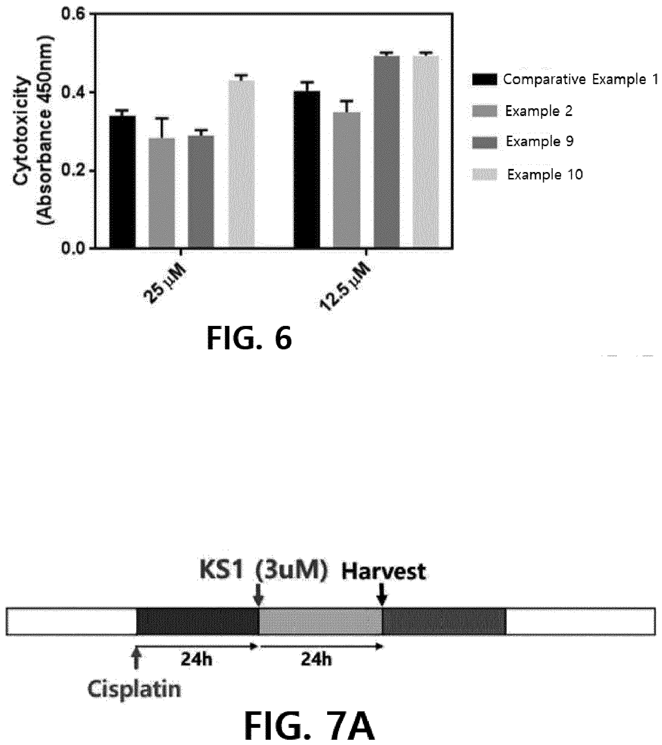

Cultured human kidney-2 (HK2) cells were treated with Comparative Example 1, Example 2 and Examples 9 to 10 at a concentration of 25 µM or 12.5 µM, and after 24 hours, cytotoxicity was measured using an EZ-Cytox kit. Since EZ-Cytox produces formazan having an absorbance at 450 nm by mitochondrial enzymes in living cells, living cells exhibit even higher absorbance at 450 nm. When the toxicity of cells treated with DMSO in the same volume as the amount of treated compound was set to 1, it was confirmed how much the cells were toxically reduced by the compound sample treatment, and the results are illustrated in FIG. 6.

As illustrated in FIG. 6, it was confirmed that when the cells were treated at a concentration of 12.5 µM or 25 µM, the compound of Example 10 showed the least toxicity, and the toxicity showed an improvement of 20% or more even when compared to Comparative Example 1.

### <Experimental Example 5> Analysis of klotho protein expression level in HK-2 cells

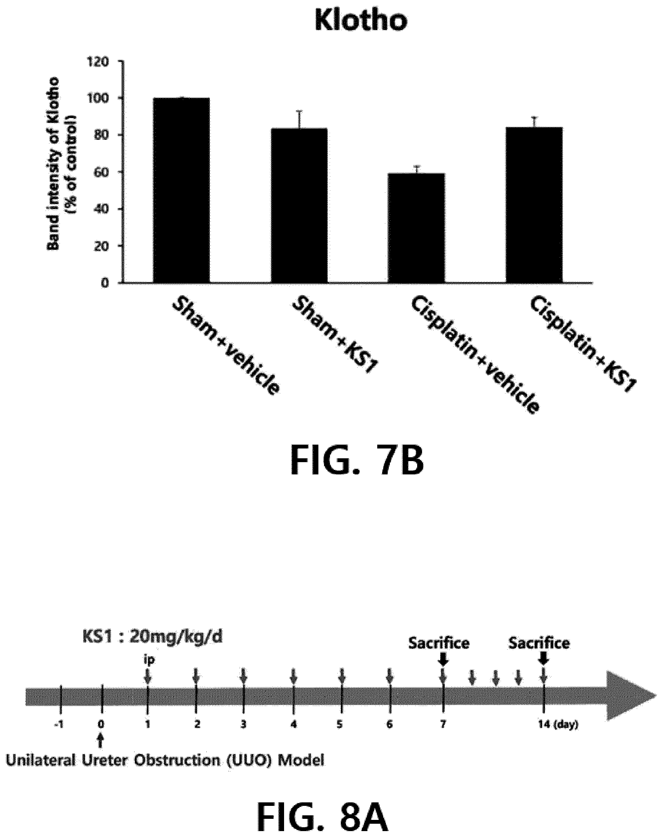

As in the protocol in FIG. 7A, a disease model was made by treating HK-2 cells (human renal cells) with cisplatin (20 µM), and then treated with a KS1 compound (Example 10) (3 µM). After 24 hours, the expression degree of the klotho protein was confirmed by obtaining the cells. As illustrated in FIG. 7B, it was confirmed that the expression of the klotho protein in HK-2 cells was decreased in a group treated with cisplatin, and was increased in a group treated with the KS1 compound (Example 10).

### <Experimental Example 6> Analysis of effect of KS1 compound (Example 10) in unilateral ureter obstruction (UUO) model

In a unilateral ureter obstruction model, 5-week-old male C56BL/6 mice were used. After the right ureter was tied with a thread to create a unilateral ureter obstruction model, the KS1 compound (20 mg/kg/day) was injected into the abdominal cavity daily starting 24 hours later. After that, samples were collected by sacrificing the mice on days 7 and 14 and tested (FIG. 8A).

### Confirmation of presence or absence of renal hypertrophy

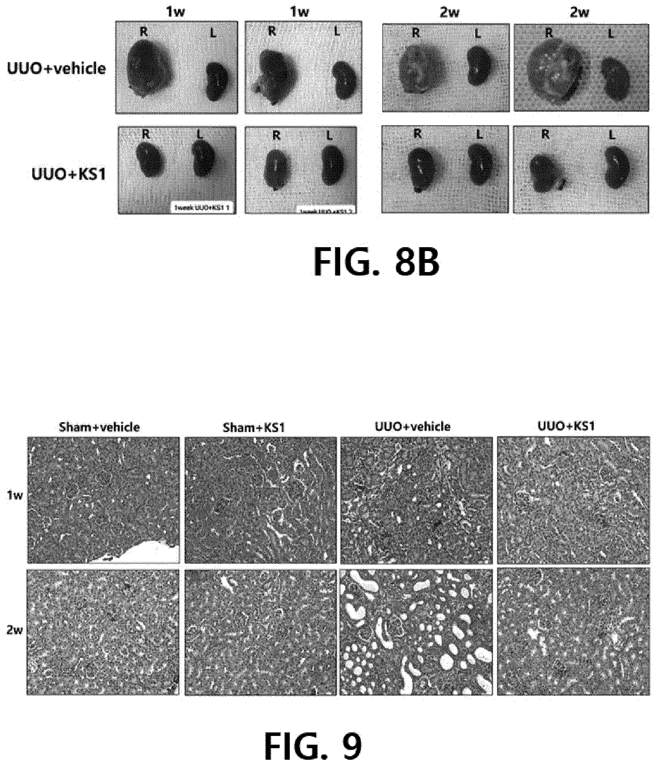

As a result of the experiment, the unilateral ureter obstruction model showed a result that the kidneys were hypertrophied, and the kidneys treated with the KS1 compound showed a result that there was no difference in size (FIG. 8B).

### Confirmation of nuclear and cytoplasmic changes

H&E staining was performed to investigate changes in the nucleus and cytoplasm in the unilateral ureter obstruction model, and the results are illustrated in FIG. 9. It could be seen that in the one-week sample of the unilateral ureter obstruction model which was not treated with KS1, the number of nuclei increased significantly, and the two-week sample showed that the cytoplasm was destroyed whereas the KS1-treated sample showed a large reduction in tissue destruction (FIG. 9).

### Confirmation of whether fibrosis progressed

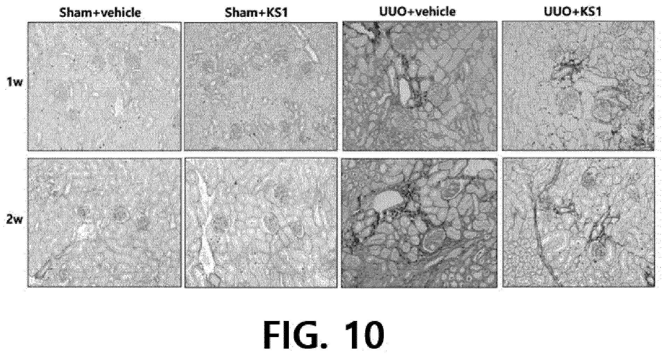

In addition, Sirius Red staining was performed to investigate the change according to the progress of fibrosis in the unilateral ureter obstruction model. In the one-week tissue sample of the unilateral ureter obstruction model that was not treated with KS1, it can be seen from the color red that fibrosis began to progress, and the two-week sample showed a result that fibrosis had progressed more than the one-week sample. However, the KS1 treatment group showed a result that fibrosis occurred less than the unilateral ureter obstruction model which was not treated with KS1 (FIG. 10).

### Confirmation of presence or absence of apoptosis

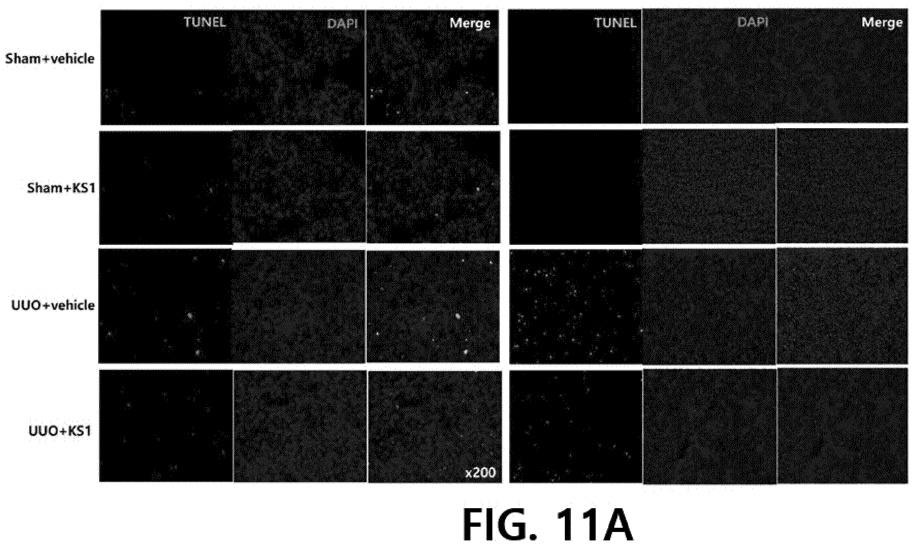

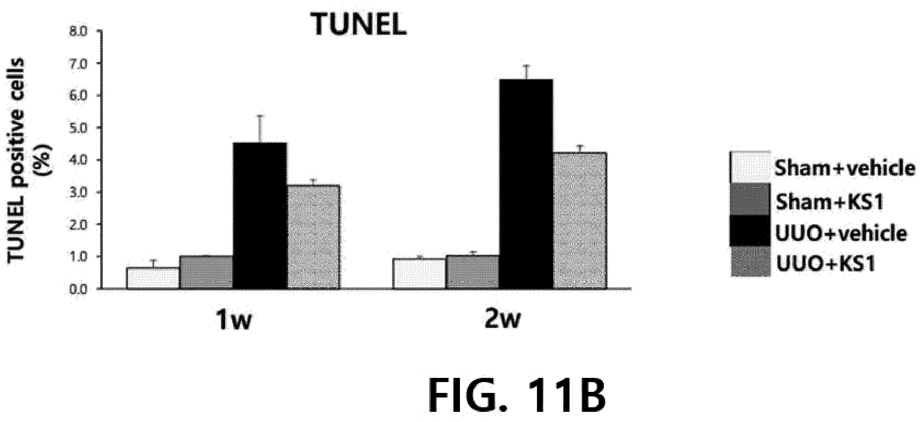

TUNEL staining was performed to investigate the degree of apoptosis in the unilateral ureter obstruction model. Apoptosis occurred in the one-week sample of the tissue of the unilateral ureter obstruction model which was not treated with KS1, and the two-week sample showed a result that apoptosis was increased compared to the one-week sample. However, the KS1 treatment group showed a result that apoptosis was remarkably reduced compared to the unilateral ureter obstruction model which was not treated with KS1 (FIG. 11).

### Confirmation of klotho protein expression level

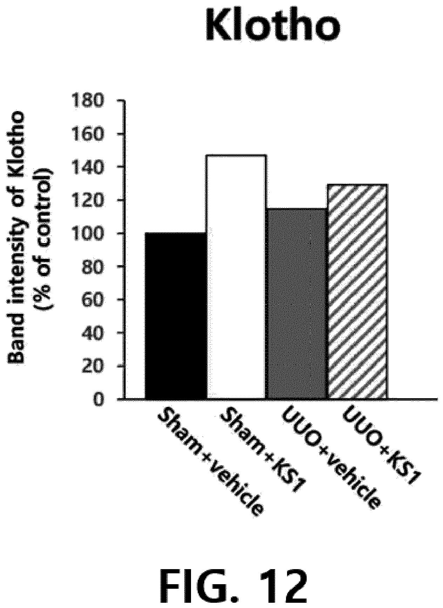

As a result of confirming the change in klotho protein expression in the one-week sample of the unilateral ureter obstruction model, klotho protein was decreased in the unilateral ureter obstruction model sample which was not treated with KS1, but the case of treatment with the KS1 compound showed a result that the expression of klotho protein was increased (FIG. 12).

### Analysis of MMP-9 protein expression level

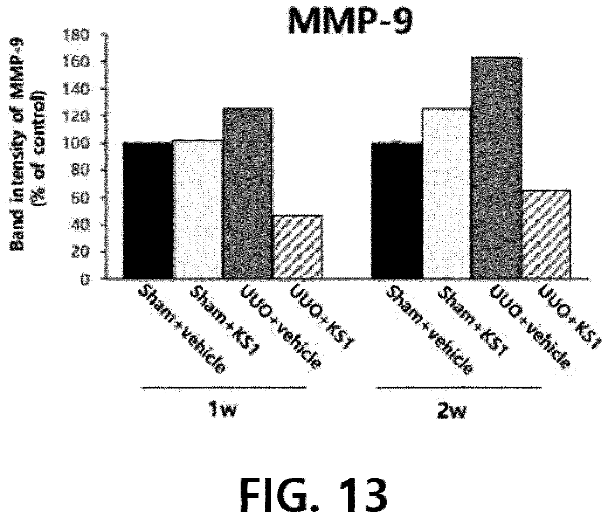

As a result of confirming the change in MMP-9 protein, which is an inflammation marker, in the unilateral ureter obstruction model, MMP-9 was increased in the samples of week 1 and week 2 of the unilateral ureter obstruction model which was not treated with KS1, and the case where the sample was treated with KS1 showed a result that the expression level of MMP-9 was remarkably reduced (FIG. 13).

### <Experimental Example 7> Analysis of effect of reducing hypertension of KS1 compound (Example 10)

### 1. Experimental rat

A 6-week-old rat male WKY/lzm was used as a normal control, and a 6-week-old rat male SHR/lzm was used as an experimental group in which hypertension was induced. All animals were purchased from the Central Laboratory Animal, and experiments were conducted at the rearing facility in a re-entry area within the Experimental Animal Center of the Osong Medical Innovation Foundation. The experimental mice were quarantined and acclimatized for 7 days after receipt, and the health of the animals was observed during this period.

### 2. Rat breeding environment

### (1) Environmental conditions

This experiment was conducted at the rearing facility in a re-entry area within the Experimental Animal Center of the Osong Medical Innovation Foundation, which was set to a temperature of 22 ± 2°C, a relative humidity of 50 ± 10%, a ventilation frequency of 10 to 15 times/hr, a lighting time of 12 hours (8 a.m. lighting on to 8 p.m. lighting off), and an illuminance of 500 Lux or more. All experimenters performed the work while wearing high-pressure steam sterilized (121°C, 20 minutes) work clothes and protective equipment.

### (2) Cage and rearing density

Test animals were reared with 2 animals/cage during the acclimatization period and the experiment period in cages (W200 X D310 X H150) according to the AAALAC guidelines in individual ventilation cage (IVC) systems.

### (3) Feed and water feeding method

For the feed supplied for the test, solid feed for test animals (+40RMM-10, SAFE) sterilized by irradiation was fed, and as a result of reviewing the component analysis report of the feed, there were no factors affecting the test in a feed composition and contaminant testing. Water obtained after sterilizing ultrapure water produced in an RO water (reverse osmosis distilled water) production and supply device through a sodium hypochlorite (NaOCl) and UV device was allowed to be freely ingested through a water bottle, and was commissioned to an accredited organization (Chungcheongbuk-do Institute of Health and Environment, 184, Osongsaengmyeong 1-ro, Osong-eup, Heungdeok-gu, Cheongju-si) and judged as 'conforming to the water quality standards for drinking water' as a result of conducting regular water quality inspections.

### (5) Quarantine and acclimatization

At the time of import, a visual inspection of animals was conducted. During the acclimatization period, general symptoms were observed once a day and recorded on a general symptom observation paper. On the end day of the acclimatization period, the health status of the animals was evaluated by checking general symptoms.

### (6) Individual and breeding box identification

During the acclimatization period, an individual was marked using a red oil pen on the tail of the animal upon receipt, and an individual identification card was attached to the breeding box during the quarantine and acclimatization period. During the observation period, when groups were separated, an individual was marked using a blue oil pen on the tail of the animal, and an individual identification card was attached to the breeding box.

### (7) Group separation

Group separation was performed on the end day of instrument acclimatization (group separation day) among animals having no general symptoms. On the day of group separation, animals were randomly divided into 5 groups with 5 animals per group such that the average blood pressure of each group was equal. The remaining animals were euthanized using CO₂.

### 3. Experimental group configuration

WKY was used as a normal control to determine whether blood pressure was elevated. The experiment was conducted by dividing SHR rats into a negative control, a test material administration group, a positive material administration group, and a test material + positive material administration group.

### 4. Preparation and administration of test materials

(1) A test material and a positive control material were dissolved in an excipient in a liquid amount of 20 mL/kg according to the corresponding concentration.
(2) After quarantine and acclimatization, the excipient, the test material, and the positive control material were administered after group separation based on the blood pressure and body weight of animals.
(3) All administration groups were administered orally 7 times/week for 8 weeks using a 3 mL syringe with a liquid amount of 20 mL/kg based on the body weight measured once a week. The concentration of the material corresponded to 20 mg/kg/day.

### 5. Blood pressure measurement

On days 36 and 64 after administration of the drug, blood pressure was measured by placing the animals in a blood pressure measuring frame and allowing the tails to come out. Blood pressure measurements were recorded using MODA High Throughput System from Kent Scientific Corporation.

### 6. Experimental results

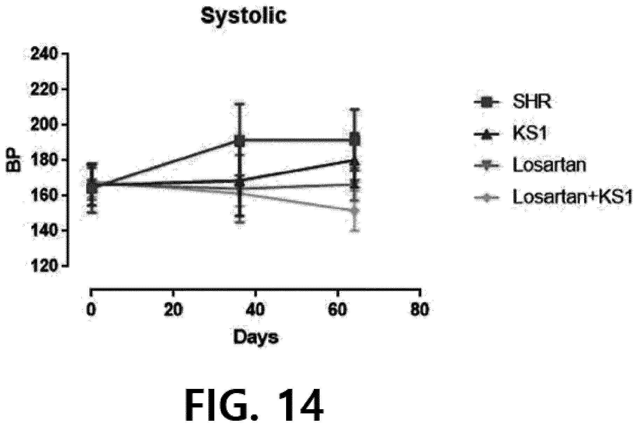

The systolic pressure of the drug-treated group measured in rats is illustrated in FIG. 14. As a result of the experiment, when a spontaneous hypertensive rat (SHR) was treated with the KS1 compound, which is an animal model of hypertension, the systolic pressure was remarkably reduced, and as a result, it can be seen that the KS1 compound can be effectively used for the prevention and treatment of hypertension. Furthermore, when co-administered with losartan, which is one of the angiotensin II receptor blocker drugs used as a therapeutic agent for hypertension, the co-administration showed a better blood pressure reduction effect than when each drug was administered independently, and it could be seen that KS 1 can also be used in co-administration for the treatment of hypertension.

### <Experimental Example 5> Analysis of effect of KS1 compound (Example 10) on inhibition of cellular senescence

### 1. Cell culture and compound treatment

Human renal proximal tubule epithelial cells (RPTECs), which are renal tubular epithelial cells, were purchased from Lonza, and as a culture medium, a REGM kit supplied by the same company was also used. Cells were cultured in a 37°C incubator under 5% CO₂ conditions. When the cells reached 80% saturation, the cells were treated with trypsin, transferred to a new culture dish, and subcultured. A compound was attached to the medium such that the final concentration was 2.5 µM, and a constant concentration was maintained by adding the compound thereto during each passage. For negative control, cells containing only DMSO as a solvent were used, and cells subcultured 9 times (#9) were used as a control in which aging did not proceed. As for the cells inducing senescence, cells subcultured 17 times (#17) in total were used.

### 2. Confirmation of aging degree

"Senescence β-galactosidase staining kit" from Cell Signaling Technology, Inc. was used to confirm the degree of senescence of cells. On the day of the experiment, a staining solution was added to the cells, and the stained cells were confirmed 8 hours later. The number of stained cells was confirmed and expressed as a ratio among the total number of cells observed under a 200-fold microscope, and the number of stained cells at three different locations per sample was counted and averaged. As the senescence of the cells progresses, the expression of the enzyme β-galactosidase increases, and therefore the stained cells may be determined as more senescent cells than the non-stained cells.

### 3. Experimental results

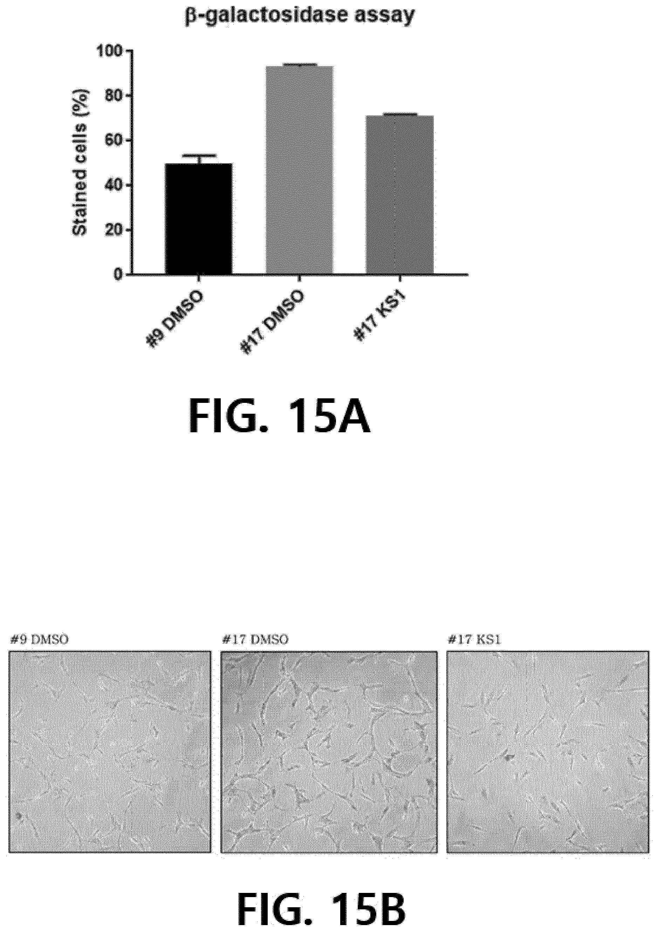

As a result of the experiment, it was found that the degree of senescence was significantly increased in the cells subcultured 17 times compared to the cells subcultured 9 times. When observed under a microscope at a magnification of 200, about 90% of the cells in the cells subcultured 17 times were all stained, but only 50% of the cells in the cells subcultured 9 times were stained (FIG. 15). In the same manner, in the cells subcultured 17 times, the degree of staining was decreased in the cell group cultured in the medium supplemented with the KS1 compound (Example 10), and therefore about 70% of the cells were stained (FIG. 15). That is, the cells cultured in the presence of the KS1 compound (Example 10) showed that the degree of senescence was decreased by about 20% compared to the cells grown in the medium supplemented with DMSO during the 17 passages.

## Claims

1. A compound represented by the following Chemical Formula 1 or a pharmaceutically acceptable salt thereof: [Chemical Formula 1] 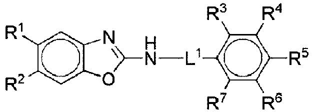 (in Chemical Formula 1, L¹ is a single bond or 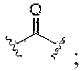 R¹ and R² are each -H, -OH, a C₁₋₁₀ straight or branched alkyl, or a C₆₋₈ arylamide, wherein the aryl of the arylamide is optionally substituted with one or more of a halogen, -NO₂ and a C₁₋₁₀ straight or branched alkyl halide; R¹ and R² optionally form a C₆₋₈ aryl with a carbon atom to which they are linked; and R³, R⁴, R⁵, R⁶ and R⁷ are each -H, a halogen, -NO₂ or a C₁₋₁₀ straight or branched alkyl).

2. The compound or the pharmaceutically acceptable salt thereof of claim 1, wherein L¹ is a single bond or 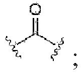 R¹ and R² are each -H, -OH, a C₁₋₅ straight or branched alkyl, or a C₆₋₇ arylamide, wherein the aryl of the arylamide is optionally substituted with one or more of a halogen, -NO₂ and a C₁₋₅ straight or branched alkyl halide; R¹ and R² optionally form a C₆₋₇ aryl with a carbon atom to which they are linked; and R³, R⁴, R⁵, R⁶ and R⁷ are each -H, a halogen, -NO₂ or a C₁₋₅ straight or branched alkyl.

3. The compound or the pharmaceutically acceptable salt thereof of claim 1, wherein L¹ is a single bond or 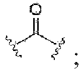 R¹ and R² are each -H, -OH, -CH₃, or phenylamide, wherein the phenyl of the phenylamide is optionally substituted with one or more of -Cl, -NO₂ and -CH₂Cl; R¹ and R² optionally form a phenyl with a carbon atom to which they are linked; and R³, R⁴, R⁵, R⁶ and R⁷ are each -H, -F, -Cl, -NO₂ or -CH₂CH₃.

4. The compound or the pharmaceutically acceptable salt thereof of claim 1, wherein L¹ is a single bond or 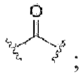 R¹ is -H, -OH, -CH₃, 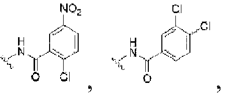 or 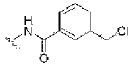 ; R² is -H; R¹ and R² optionally form a phenyl with a carbon atom to which they are linked; R³ is -H or -Cl; R⁴ is -H, -F or -Cl; R⁵ is -F, -Cl, -NO₂, or - CH₂CH₃; R⁶ is -H; and R⁷ is -H.

5. The compound or the pharmaceutically acceptable salt thereof of claim 1, wherein the compound represented by Chemical Formula 1 is any one selected from the following compound group: 1) N-(benzo[d]oxazol-2-yl)-2-chloro-4-nitrobenzamide; 2) 8-methyl-2-[N-(3,4-dichlorophenyl)]aminobenzoxazole; 3) 2-((3,4-dichlorophenyl)amino)benzo[d]oxazol-5-ol; 4) N-(2-(4-ethylphenylamino)benzo[d]oxazol-5-yl)-2-chloro-5-nitrobenzamide; 5) N-(2-(4-ethylphenylamino)benzo[d]oxazol-5-yl)-3,4-dichlorobenzamide; 6) N-(2-(4-ethylphenylamino)benzo[d]oxazol-5-yl)-3-(chloromethyl)benzamide; 7) 2-[N-(3,4-dichlorophenyl)]aminobenzoxazole; 8) N-(3,4-dichlorophenyl)naphtho[2,3-d]oxazol-2-amine; 9) N-(3,4-difluorophenyl)-5-methylbenzo[d]oxazol-2-amine; and 10) N-(3,4-difluorophenyl)benzo[d]oxazol-2-amine.

6. A method for preparing a compound represented by Chemical Formula 1A, as shown in the following Reaction Scheme 1, the method comprising: obtaining Compound 4 by dissolving Compound 2 in an organic solvent, and then adding Compound 3 thereto, and reacting the resulting mixture at 10 to 50°C for 12 to 20 hours (Step 1); and obtaining Compound 1A by adding an organic solvent in which Compound 4 obtained in Step 1 is dissolved dropwise to an organic solvent in which potassium superoxide is dissolved, and then reacting the resulting mixture at 15 to 30°C for 10 to 16 hours (Step 2): [Reaction Scheme 1] 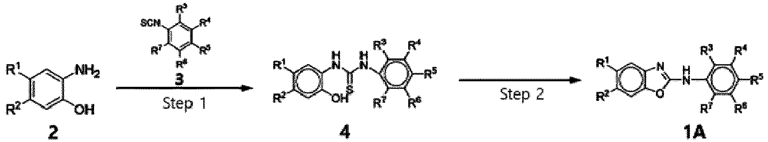 (in Reaction Scheme 1, R¹, R², R³, R⁴, R⁵, R⁶ and R⁷ are the same as defined in Chemical Formula 1 of claim 1, and Compound 1A is comprised in Chemical Formula 1 of claim 1).

7. A method for preparing a compound represented by Chemical Formula 1B, as shown in the following Reaction Scheme 2, the method comprising: obtaining Compound 6 by dissolving Compound 5 in an organic solvent, and then adding Compound 3 thereto, and reacting the resulting mixture at 10 to 50°C for 12 to 20 hours (Step 1); obtaining Compound 7 by adding an organic solvent in which Compound 6 obtained in Step 1 is dissolved dropwise to an organic solvent in which potassium superoxide is dissolved, and then reacting the resulting mixture for 12 to 24 hours (Step 2); and obtaining Compound 1B by dissolving Compound 7 in an organic solvent, adding boron tribromide (BBr₃) thereto, and then reacting the resulting mixture at room temperature for 20 to 28 hours (Step 3): [Reaction Scheme 2] 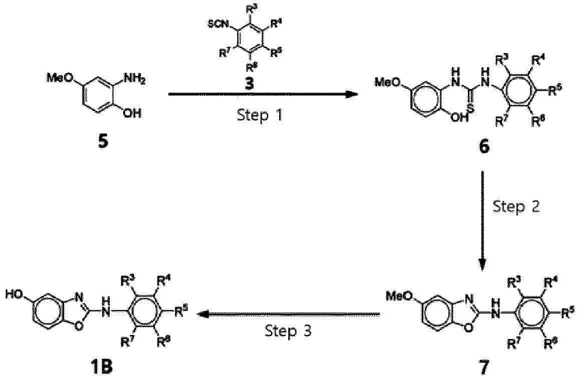 (in Reaction Scheme 2, R³, R⁴, R⁵, R⁶ and R⁷ are the same as defined in Chemical Formula 1 of claim 1, and Compound 1B is comprised in Chemical Formula 1 of claim 1).

8. A method for preparing a compound represented by Chemical Formula 1C, as shown in the following Reaction Scheme 3, the method comprising: obtaining Compound 9 by dissolving Compound 8, carbon disulfide, iodomethane and sodium hydride in an organic solvent, and then reacting the resulting solution at 10 to 50°C for 2 to 8 hours (Step 1); and obtaining Compound 1C by dissolving Compound 9 and Compound 2 in an organic solvent, and then reacting the resulting solution for 2 to 8 hours (Step 2): [Reaction Scheme 3] 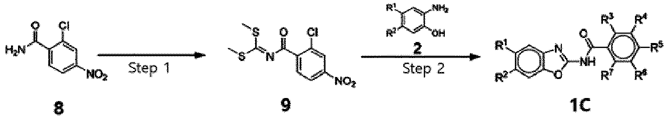 (in Reaction Scheme 3, R¹, R², R³, R⁴, R⁵, R⁶ and R⁷ are the same as defined in Chemical Formula 1 of claim 1, and Compound 1C is comprised in Chemical Formula 1 of claim 1).

9. A method for preparing a compound represented by Chemical Formula ID, as shown in the following Reaction 4, the method comprising: obtaining Compound 11 by dissolving Compound 10 and Compound 3 in an organic solvent, and then reacting the resulting solution at 10 to 50°C for 20 to 28 hours (Step 1); obtaining Compound 12 by adding an organic solvent in which Compound 11 obtained in Step 1 is dissolved dropwise to an organic solvent in which potassium superoxide is dissolved, and then reacting the resulting mixture at 10 to 50°C for 12 to 24 hours (Step 2); obtaining Compound 13 by adding Compound 12 together with a catalyst to an organic solvent, and then injecting hydrogen gas thereinto, and reacting the resulting mixture at 10 to 50°C for 12 to 20 hours (Step 3); and obtaining Compound ID by dissolving Compound 13 and Compound 14 in an organic solvent, and then reacting the resulting solution at 10 to 50°C for 12 to 24 hours (Step 4): [Reaction Scheme 4] 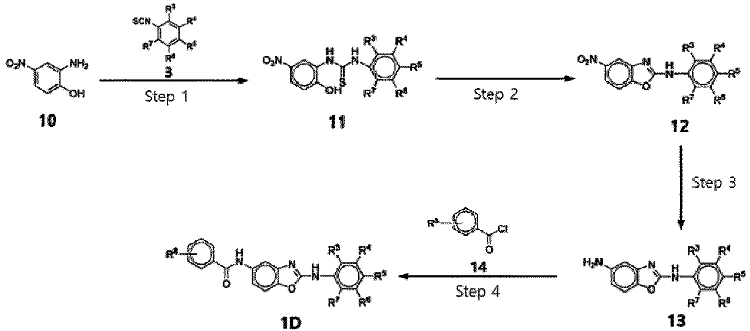 (in Reaction Scheme 4,, R³, R⁴, R⁵, R⁶ and R⁷ are the same as defined in Chemical Formula 1 of claim 1; R⁸ is one or more of a halogen, -NO₂ and a C₁₋₁₀ straight or branched alkyl halide; and Compound ID is comprised in Chemical Formula 1 of claim 1).

10. The method of any one of claims 6 to 9, wherein the organic solvent is one or more selected from the group consisting of methanol (MeOH), dimethylformamide (DMF), acetonitrile (MeCN), tetrahydrofuran (THF), dichloromethane (DCM), 1,2-dimethoxyethane, benzene, toluene, xylene, dimethyl sulfoxide (DMSO), and dioxane.

11. A composition for inhibiting cellular senescence, comprising the compound represented by Chemical Formula 1 or the pharmaceutically acceptable salt thereof of claim 1.

12. The composition of claim 11, wherein the cells are renal proximal tubule epithelial cells.

13. The composition of claim 11, wherein the composition increases the expression level of a klotho gene.

14. A cosmetic composition for preventing or alleviating skin aging, comprising the compound represented by Chemical Formula 1 or the pharmaceutically acceptable salt thereof of claim 1.

15. A health functional food composition for preventing or alleviating skin aging, comprising the compound represented by Chemical Formula 1 or the pharmaceutically acceptable salt thereof of claim 1.

16. A health food composition for preventing or alleviating skin aging, comprising the compound represented by Chemical Formula 1 or the pharmaceutically acceptable salt thereof of claim 1.

17. A pharmaceutical composition for preventing or treating a vascular aging-induced disease, comprising the compound represented by Chemical Formula 1 or the pharmaceutically acceptable salt thereof of claim 1.

18. A pharmaceutical composition for preventing or treating a vascular aging-induced disease, comprising the compound represented by Chemical Formula 1 or the pharmaceutically acceptable salt thereof of claim 1, and an angiotensin II receptor blocker.

19. A health functional food composition for preventing or treating a vascular aging-induced disease, comprising the compound represented by Chemical Formula 1 or the pharmaceutically acceptable salt thereof of claim 1.

20. A health food composition for preventing or treating a vascular aging-induced disease, comprising the compound represented by Chemical Formula 1 or the pharmaceutically acceptable salt thereof of claim 1.

21. The composition of any one of claims 17 to 20, wherein the vascular aging-induced disease is selected from the group consisting of hypertension, angina pectoris, myocardial infarction, arteriosclerosis, prostate hyperplasia, vasospasm contraction, thrombosis, cerebral infarction and cerebral hemorrhage.

22. A pharmaceutical composition for preventing or treating a renal disease, comprising the compound represented by Chemical Formula 1 or the pharmaceutically acceptable salt thereof of claim 1.

23. A health functional food composition for preventing or alleviating a renal disease, comprising the compound represented by Chemical Formula 1 or the pharmaceutically acceptable salt thereof of claim 1.

24. A health food composition for preventing or alleviating a renal disease, comprising the compound represented by Chemical Formula 1 or the pharmaceutically acceptable salt thereof of claim 1.

25. The composition of any one of claims 23 and 24, wherein the renal disease is selected from the group consisting of nephritis, pyelitis, nephrotic syndrome, acute pyelonephritis, chronic pyelonephritis, urinary tract infection, diabetic nephropathy, chronic glomerulonephritis, acute progressive nephritis, nephrotic syndrome, microglomerular sclerosis, membranous nephropathy or membranoproliferative glomerulonephritis.


<110>    Klotho Sciences

<120>    Compounds that induces anti-aging gene klotho and use thereof

<130>    PCT5040613

<150>    KR 10-2019-0122305
<151>    2019-10-02

<160>    4

<170>    KoPatentIn 3.0

<210>    1
<211>    24
<212>    DNA
<213>    Artificial Sequence

<220>
<223>    KL-F

<400>    1
gatagagaaa aatggcttcc ctcc                                              24

<210>    2
<211>    24
<212>    DNA
<213>    Artificial Sequence

<220>
<223>    KL-R

<400>    2
ggtcggtaaa ctgagacaga gtgg                                              24

<210>    3
<211>    24
<212>    DNA
<213>    Artificial Sequence

<220>
<223>    GAPDH-F

<400>    3
tgacaacttt ggtatcgtgg aagg                                              24

<210>    4
<211>    22
<212>    DNA
<213>    Artificial Sequence

<220>
<223>    GAPDH-R

<400>    4
agggatgatg ttctggagag cc                                                22